(19) Europäisches Patentamt
European Patent Office
Office européen des brevets

(11) EP 4 609 942 A1

(12) EUROPÄISCHE PATENTANMELDUNG

(43) Veröffentlichungstag:
03.09.2025 Patentblatt 2025/36

(21) Anmeldenummer: 25160111.8

(22) Anmeldetag: 25.02.2025

(51) Internationale Patentklassifikation (IPC):
B01D 61/08 (2006.01)      B01D 61/12 (2006.01)

(52) Gemeinsame Patentklassifikation (CPC):
B01D 61/08; B01D 61/12; C02F 1/441;
B01D 2311/24; B01D 2311/243; B01D 2311/251;
B01D 2313/70; B01D 2313/701; C02F 2209/05

(84) Benannte Vertragsstaaten:
AL AT BE BG CH CY CZ DE DK EE ES FI FR GB
GR HR HU IE IS IT LI LT LU LV MC ME MK MT NL
NO PL PT RO RS SE SI SK SM TR
Benannte Erstreckungsstaaten:
BA
Benannte Validierungsstaaten:
GE KH MA MD TN

(30) Priorität: 29.02.2024 DE 102024105880

(71) Anmelder: B. Braun Avitum AG
34212 Melsungen (DE)

(72) Erfinder:
• LIEB, Nino
  34212 Melsungen (DE)
• ODERNHEIMER, Philipp
  34212 Melsungen (DE)

(74) Vertreter: Tergau & Walkenhorst
Intellectual Property GmbH
Lurgiallee 12
60439 Frankfurt am Main (DE)

(54) UMKEHROSMOSEANLAGE

(57) Vorrichtung zur Überwachung von Güteparametern von Umkehrosmose-Prozessflüssigkeiten einer Umkehrosmoseanlage umfassend

- eine Steuerungseinheit mit wenigstens einem ersten Prozessor und einem zweiten Prozessor,

- eine Kommunikationsschnittstelle eingerichtet zum Datentransfer zwischen dem ersten Prozessor und dem zweiten Prozessor,

- wobei der erste Prozessor eingerichtet ist zur Erfassung und Verarbeitung eines durch einen ersten Güteparametersensor detektierten ersten Sensorwertes eines ersten Güteparameters eines Permeats der Umkehrosmose-Prozessflüssigkeiten,

- wobei der zweite Prozessor eingerichtet ist zur Erfassung und Verarbeitung eines durch wenigstens einen weiteren Güteparametersensor detektierten zweiten Sensorwertes des ersten Güteparameters des Permeats,

- wobei die Steuerungseinheit eingerichtet ist zum Berechnen einer Abweichung des ersten Sensorwertes von dem zweiten Sensorwert und zum Vergleichen des ersten Sensorwertes und/oder des zweiten Sensorwerts mit einem vorbestimmbaren Grenzwert des ersten Güteparameters, und

- wobei die Steuerungseinheit eingerichtet ist zur Steuerung der Pumpenleistung wenigstens einer ersten Pumpe oder wenigstens einer weiteren Pumpe der Umkehrosmoseanlage,

- wobei der erste Güteparameter bevorzugt die Leitfähigkeit des Permeats ist.

Fig. 3B

**Beschreibung**

**Technisches Gebiet**

Die vorliegende Erfindung betrifft eine Vorrichtung zur Überwachung von Güteparametern von Umkehrosmose-Prozessflüssigkeiten einer Umkehrosmoseanlage, eine Umkehrosmoseanlage, ein Verfahren zur Überwachung von Güteparametern von Umkehrosmose-Prozessflüssigkeiten einer Umkehrosmoseanlage, sowie ein Computerprogramm und das Computerprogramm umfassendes Speichermedium.

**Hintergrund**

Umkehrosmoseanlagen sind als Anlagen spezieller Filtrations- oder Trenntechnologien bekannt und basieren auf dem Prinzip der Umkehrosmose.

DE202024101009 U1 betrifft eine Umkehrosmoseanlage. Die Umkehrosmose ist ein physikalisches Verfahren, bei dem Wasser durch eine halbdurch-lässige Membran gepresst wird, um Verunreinigungen, insbesondere gelöste Fest-stoffe, Ionen und Moleküle zu entfernen. Die Membran erlaubt im Wesentlichen nur dem Wasser, durch die Membran zu gelangen, während größere Partikel zurückgehalten werden.

In der medizinischen Anwendung, insbesondere bei der Dialyse, wird die Umkehrosmoseanlage für die Wasseraufbereitung verwendet. Bei der Dialyse wird das Blut eines Patienten außerhalb des Körpers durch eine Maschine geleitet, die die Funktionen der Nieren übernimmt, indem sie Abfallstoffe und überschüssige Flüssigkeit entfernt. Da Wasser ein wichtiger Bestandteil dieses Prozesses ist, muss das Wasser extrem rein sein, um Komplikationen für den Patienten zu vermeiden.

Die Umkehrosmoseanlage spielt dabei eine entscheidende Rolle, indem sie das Prozesseingangswasser, bzw. ggf. auch nicht entnommenes Permeat aus der Ringleitung, durch die Membran bzw. die Membranen leitet, um Verunreinigungen zu eliminieren. Das resultierende gereinigte Wasser wird dann im Dialysegerät verwendet, um das Blut des Patienten zu reinigen.

Durch den Einsatz von Umkehrosmose wird sichergestellt, dass potenziell schädliche Substanzen, wie Bakterien, Viren, Chemikalien und andere Verunreinigungen, aus dem Wasser entfernt werden. Dies ist entscheidend, um eine sichere und effektive Durchführung der Dialyse zu gewährleisten und das Risiko von Infektionen oder anderen Komplikationen zu minimieren.

Dabei ist es im Stand der Technik üblich, die Funktionsfähigkeit der Umkehrosmoseanlage und Güteparametern von Umkehrosmose-Prozessflüssigkeiten, insbesondere die Güte des Permeats, also nach Filtrierung durch die Membran, zu überwachen. Dies kann insbesondere durch die Überwachung der Leitfähigkeit des Permeats mittels eines Leitfähigkeitssensors. Die Detektion des Sensorwertes der Leitfähigkeit wird über eine Steuerungseinheit, welche üblicherweise innerhalb der Umkehrosmoseanlage angebracht ist, durchgeführt. Sollte die Steuerungseinheit über den Leitfähigkeitssensor einen Anstieg über einen festgelegten Grenzwert registrieren, wird die Umkehrosmoseanlage gestoppt. Der Stoppvorgang wird dadurch realisiert, dass die für die Förderung der Umkehrosmose-Prozessflüssigkeiten vorgesehenen Pumpen, z.B. eine Druckpumpe und eine Zirkulationspumpe, über die Steuerungseinheit gestoppt wird.

Dabei besteht das Problem, dass bei Ausfall oder Störung der Sensorik oder einem Softwarefehler der Steuerungseinheit keine Ausfallkompensation gegeben ist und insbesondere keine Redundanz in der Pumpenabschaltung gegeben ist und ggf. keine Möglichkeit besteht, die Anlage zu stoppen. Selbst beim Schaffen einer Redundanz, insbesondere beim Bereitstellen einer zweiten Steuerungseinheit kann die fehlende Zustandskontrolle und Rücksetzungsmaßnahme nicht erreicht werden. Dabei kommt es in im Stand der Technik bekannten Geräten zu falsch positiven Alarmen bei chemischer und thermischer Desinfektion, insbesondere, da die Leitfähigkeiten des Permeats bei diesen Betriebsphasen über die erlaubten Grenzwerte steigen können und somit eine Abschaltung ungewollt ist. Daneben können auch längere Standzeiten, bspw. in einem Stand-by-Betrieb, zu einem falsch positiven Abschalten führen, was eine Wiederinbetriebnahme der Anlage deutlich erschwert.

**Zusammenfassende Beschreibung der Erfindung**

Es ist deshalb eine der vorliegenden Erfindung zu Grunde liegende Aufgabe die Nachteile von Umkehrosmoseanlagen im Stand der Technik zu überwinden. Insbesondere ist es eine der vorliegenden Erfindung zu Grunde liegende Aufgabe, Umkehrosmoseanlagen bereitzustellen, die über eine verbesserte Ausfallkompensation, Sicherheit und/oder eine verbesserte Zustandskontrolle verfügen.

Diese und andere Probleme werden durch den Gegenstand der beigefügten unabhängigen Ansprüche gelöst.

Bevorzugte Ausführungsformen können den abhängigen Ansprüchen entnommen werden und darüber hinaus aus der folgenden Beschreibung, insbesondere unter Berücksichtigung verschiedener Ausführungsformen, wie sie in den beigefügten Ansprüchen behandelt und beschrieben sind.

Die Ausführungsformen, Merkmale und Kombinationen von Merkmalen, wie sie hier in Verbindung mit der Erfindung beschrieben werden, sowie die Kombination von Merkmalen, wie sie in den beigefügten Ansprüchen angegeben sind, aber auch jede Kombination von Merkmalen, wie sie in Verbindung mit den Ausführungsformen erwähnt und beschrieben wird, gelten als hierin offenbart, zumindest jedoch als durch einen Fachmann herleitbar. Insbesondere kann jedes Merkmal und jede Kombination von Merkmalen in den hier beschriebenen Ausführungsformen beispielsweise in einer anderen Kombination, insbesondere in einer anderen Anspruchskategorie, beansprucht werden, mindestens weil die fachkundige Person erkennen wird, dass jede einzelne Kombination der hier genannten Merkmale geeignet ist, zur Lösung des zugrunde liegenden Problems beizutragen.

Des Weiteren können jedes Merkmal und jede Kombination von Merkmalen in den Ansprüchen und in der untenstehenden Beschreibung unabhängig von dem jeweils beanspruchten Gegenstand, unabhängig von Anspruchsabhängigkeiten und Rückverweisen sowie unabhängig von der Anspruchskategorie, in der das Merkmal beansprucht wird, verwendet und separat beansprucht werden. Beispielsweise kann in einer willkürlichen Kombination, die aus einem oder mehreren Ansprüchen ausgewählt ist, eine oder mehrere Ausführungsformen gemäß der untenstehenden Beschreibung und/oder den beigefügten Abbildungen vorgesehen sein.

Die oben beschriebenen Aufgaben werden erfindungsgemäß gelöst durch eine Vorrichtung zur Überwachung von Güteparametern von Umkehrosmose-Prozessflüssigkeiten einer Umkehrosmoseanlage. Eine solche Vorrichtung umfasst eine Steuerungseinheit mit wenigstens einem ersten Prozessor und einem zweiten Prozessor. Eine derartige Bereitstellung von wenigstens zwei Prozessoren einer Steuerungseinheit ist besonders vorteilhaft, um eine Redundanz zu schaffen, und eine verbesserte Zustandskontrolle zu erreichen. Insbesondere bei Ausfall eines der Prozessoren, kann der zweite Prozessor zum einen (Teil-)Aufgaben des ausgefallenen Prozessors übernehmen und/oder den Zustand des anderen Prozessors überprüfen bzw. detektieren. Dabei kann die Software des zweiten Prozessors, ggf. Kontrollprozessors, im Vergleich zu der des ersten Prozessors, ggf. Hauptprozessors, vorteilhaft einfach ausgestaltet werden. Dies erlaubt einen geringeren Programmieraufwand, Speicherplatzbedarf, Hardwareaufbau oder ähnliches und ist auch unter regulatorischen Gesichtspunkten, bspw. Risikobetrachtung, Testdurchführung etc., ggf. sinnvoll. Dies ist der Fall, da die Aufgaben, welche vom Kontrollsystem ausgeführt werden sollen, zumeist risikominimierender Natur sein werden. Dabei kann es vorteilhaft sein, dass ein Prozessor, bevorzugt der erste Prozessor, die Funktion eines Hauptprozessors (Main) übernimmt und ein zweiter Prozessor die Funktion eines Nebenprozessors übernimmt. Die erfindungsgemäße Vorrichtung umfasst weiter eine Kommunikationsschnittstelle, die eingerichtet ist zum Datentransfer zwischen dem ersten Prozessor und dem zweiten Prozessor. Über eine derartige Kommunikationsschnittstelle können die Prozessoren der Vorrichtung Daten austauschen. Dabei arbeiten die beiden Prozessoren bevorzugt voneinander unabhängig, können jedoch auch über die Kommunikationsschnittstellen Datenpakete austauschen. Die Kommunikationsschnittstelle ist dabei bevorzugt bidirektional ausgestaltet, so dass eine Übertragung von Daten zwischen den Prozessoren in beiden Richtungen erfolgen kann. In einer bevorzugten Ausgestaltung sind die Prozessoren in Ihrem Funktions- und Leistungsumfang im Wesentlichen ähnlich, aber baulich unterschiedlich. Dabei erweist es sich nach Überzeugung der vorliegenden Erfinder als "Best Practice", wenn zwei nicht baugleiche/identische Prozessoren in der vorliegenden Erfindung als erster und zweiter Prozessor verwendet werden, da hierdurch ggf. das Risiko eines simultanen Ausfalls bzw. eines systematischen, ggf. bauseitigen Bauserien-, Fehlers möglichst verringert wird. Gemäß der vorliegenden Erfindung ist der erste Prozessor, der bevorzugt als Hauptprozessor dient, dazu eingerichtet einen durch einen ersten Güteparametersensor detektierten ersten Sensorwertes eines ersten Güteparameters eines Permeats der Umkehrosmose-Prozessflüssigkeit zu erfassen und zu verarbeiten. Ein solcher Güteparameter ist vorzugsweise ein sicherheitsrelevanter Parameter im erzeugten Permeat, beispielsweise die Leifähigkeit des Permeats. Der zweite Prozessor ist eingerichtet einen weiteren, zweiten Sensorwert des ersten Güteparameters des Permeats, der durch wenigstens einen weiteren Güteparametersensor detektiert wird zu erfassen und zu verarbeiten. Durch die redundante Detektion des Güteparameters mittels zweier redundanter Güteparametersensoren und der unabhängigen und redundanten Übermittlung und Verarbeitung der Sensordaten durch die beiden Prozessoren ist die Betriebssicherheit der Umkehrosmoseanlage mit der erfindungsgemäßen Vorrichtung vorteilhaft verbessert. Insbesondere die unabhängigen voneinander erfassten Leitfähigkeiten eines Permeats als Güteparameter kann dabei vorteilhaft zur Erhöhung der Sicherheit der erfindungsgemäßen Umkehrosmoseanlage beitragen, sowie deren Einsatzfähigkeit dadurch erhöhen, dass die Güte der Umkehrosmose-Prozessflüssigkeiten für den bestimmungsgemäßen Einsatz sichergestellt werden kann. Das bedeutet, dass sich zunächst die erfindungsgemäße Vorrichtung vorteilhafterweise, dazu eignet die Überwachung der Güte der Umkehrosmose-Prozessflüssigkeiten zu verbessern. Als eine Regelmaßnahme kann dabei eine Statusmeldung über den Zustand der Umkehrosmoseanlage und/oder der Umkehrosmose-Prozessflüssigkeiten an einen Benutzer ausgegeben werden. Weiterhin kann sodann die Güte der Umkehrosmose-Prozessflüssigkeiten verbessert bzw. sichergestellt werden, indem entsprechende Regelmaßnahmen, insbesondere das Steuern einer physikalischen Maßnahme, bspw. einer Pumpe oder eines Ventils, durch die Steuereinheit entsprechend veranlasst werden. Die Vornahmen einer Regelmaßnahme wird insbesondere basierend auf der Verarbeitung der Sensorwerte mittels der Steuereinrichtung entschieden. Die erfindungsgemäße Vorrichtung ist dabei derart ausgestaltet, dass die Steuerungseinheit zum Berechnen einer Abweichung des ersten Sensorwertes von dem zweiten Sensorwert eingerichtet ist. Zusätzlich oder alternativ wird der erste Sensorwerte und/oder der zweite Sensorwert mit einem vorbestimmbaren Grenzwert des ersten Güteparameters verglichen.

Der Begriff "Güteparameter", wie hierin verwendet, bezeichnet bevorzugt einen physikalischen Parameter einer Flüssigkeit, der indikativ oder maßgebend für die Güte und Qualität der Flüssigkeit ist. Dieser Parameter kann verschiedene Aspekte der Flüssigkeit charakterisieren, die sich auf ihre Brauchbarkeit, Reinheit oder andere qualitative Eigenschaften beziehen. Beispiele für Güteparameter könnten Leitfähigkeit, Temperatur, Druck, pH-Wert, Konzentration bestimmter Stoffe, Trübheit, Viskosität oder andere messbare Eigenschaften der Flüssigkeit sein, die auf ihre Zusammensetzung und Eignung für bestimmte Zwecke hinweisen. Der Fachmann wird unmittelbar anerkennen, dass in der medizinischen Anwendung der Vorrichtung und/oder Umkehrosmoseanlage nach der vorliegenden Erfindung die Überwachung und Kontrolle von Güteparametern der Umkehrosmose-Prozessflüssigkeiten entscheidend ist, um sicherzustellen, dass die Flüssigkeit den erforderlichen Standards und Anforderungen entspricht.

Die erfindungsgemäße Vorrichtung zur Überwachung der Umkehrosmoseanlage ist dabei insbesondere Teil einer erfindungsgemäßen Umkehrosmoseanlage, durch die die oben beschriebenen Aufgaben ebenfalls erfindungsgemäß gelöst werden können.

Die Umkehrosmoseanlage gemäß der vorliegenden Erfindung ist dabei insbesondere eine Umkehrosmoseanlage zu medizinischen Zwecken, die geeignet ist um an ein medizinisches System, insbesondere wenigstens ein medizinisches Endgerät, insbesondere ein Dialysegerät angeschlossen zu werden und diese mit Permeat zu versorgen.

Der Fachmann auf dem Gebiet der medizinischen Umkehrosmose weiß, dass die Umkehrosmoseanlagen insbesondere an sogenannte Ringleitungen angeschlossen werden können. Eine derartige im Stand der Technik bekannte Ringleitung ist bspw. eine Versorgungsleitung innerhalb eines nephrologischen Zentrums (zum Beispiel einem Dialysezentrum, Krankenhaus etc.) an dem eine Vielzahl an Dialysemaschinen bzw. Dialysegeräten angeschlossen werden können.

Eine solche Umkehrosmoseanlage umfasst einen Vorlagetank, der über eine Prozesseingangswasserzuführung mit Prozesswasser versorgbar ist und weiter eine Rücklauf-Zuführung umfasst, mittels der Rücklaufflüssigkeit aus einer mit wenigstens einem medizinischen Endgerät, insbesondere einem Dialysegerät, verbundenen Ringleitung in den Vorlagetank einspeisbar ist. Unter dem Begriff "Rücklaufflüssigkeit", wie hierin verwendet, wird in diesem Zusammenhang bevorzugt verstanden, dass im Wesentlichen nicht verbrauchtes und nicht aus der Ringleitung entnommenes Permeat aus der Ringleitung zurückgeführt wird. Die Umkehrosmoseanlage umfasst dabei wenigstens eine erste Filter-Pumpen-Einheit, mit wenigstens einer Pumpe, insbesondere einer Druckpumpe, die fluidisch mit dem Vorlagetank verbunden ist. Die erste Pumpe, bevorzugt eine Druckpumpe, ist so angeordnet, dass die Membran mittels der Pumpe mit Umkehrosmose-Prozessflüssigkeiten aus dem Vorlagetank zur Erzeugung des Permeats beaufschlagt werden kann. Hierdurch wird das Permeat als Produkt und ein Konzentrat als Nebenprodukt aus der Umkehrosmose-Prozessflüssigkeit des Vorlagetanks erzeugt. Weiter umfasst die Umkehrosmoseanlage eine mit der wenigstens einen Membran fluidisch verbunden Permeatzuleitung, so dass das erzeugte Permeat über die Ringleitung wenigstens einem medizinischen Endgerät, insbesondere einem Dialysegerät, zuführbar ist. Es soll verstanden werden, dass die erfindungsgemäße Umkehrosmoseanlage damit bevorzugt in ein Kreislaufsystem, bevorzugt mit einer solchen Ringleitung, integriert ist und zum einen Permeat zum Betreiben der medizinischen Endgeräte, insbesondere Dialysegeräte, dem Kreislauf, und insbesondere einer mit dem wenigstens einen medizinischen Endgerät, insbesondere einem Dialysegeräts, verbundenen Ringleitung, bereitstellt, und zum anderen aus dem Kreislauf, insbesondere aus einer mit dem wenigstens einen medizinischen Endgerät, insbesondere einem Dialysegeräts, verbundenen Ringleitung, nicht verbrauchtes Permeat über den Rücklauf zurück in den Vorlagetank gelangt.

Zusätzlich kann die Umkehrosmoseanlage wenigstens eine weitere Pumpe, insbesondere eine Zirkulationspumpe umfassen, die, um Wasser zu sparen, einen Teil des Konzentrats in den Prozess wiedereingespeist, und insbesondere der in einer Zuleitung zur Membran befindlichen Umkehrosmose-Prozessflüssigkeit aus dem Vorlagetank zuleitet. Vorteilhafterweise ist die Steuerungseinheit dazu eingerichtet die Pumpenleistung der ersten Pumpe und der zweiten Pumpe voneinander unabhängig zu steuern. So kann insbesondere die Zuführung von Prozessflüssigkeit zur Membran und alternativ oder zusätzlich die Zirkulation von Konzentrat geregelt werden.

Durch die Aufkonzentrierung von Stoffen im gesamten Kreislauf des Systems, ist es insbesondere notwendig, die Güte der Prozessflüssigkeit und insbesondere des Permeats zu überwachen. Hierzu ist in der erfindungsgemäßen Umkehrosmoseanlage ein erste Güteparametersensor zum Detektieren eines ersten Sensorwertes eines ersten Güteparameters des Permeats der Umkehrosmose-Prozessflüssigkeiten vorgesehen. Zusätzlich ist wenigstens ein weiterer Güteparametersensor zum Detektieren eines zweiten Sensorwertes des ersten Güteparameters des Permeats der Umkehrosmose-Prozessflüssigkeiten vorgesehen. So kann der erste Güteparameter, bevorzugt die Leitfähigkeit, des Permeats als Güteparameter mehrfach und voneinander unabhängig detektiert werden.

Die Umkehrosmoseanlage nach der vorliegenden Erfindung umfasst bevorzugt auch eine Ableitung für Konzentrat und ein mit der Ableitung verbundenes Ablassventil. Bei einer erfindungsgemäßen Vornahme einer Regelmaßnahme, kann dieses Ventil geöffnet werden, um Konzentrat aus dem System abzuleiten. Das Volumen der abgeleiteten Flüssigkeit kann dabei erfindungsgemäß mittels der Erfassung und Verarbeitung eines durch einen ersten Güteparametersensor detektierten ersten Sensorwertes eines ersten Güteparameters eines Permeats der Umkehrosmose-Prozessflüssigkeiten, über das so festgestellte Gütemaß geregelt werden. Es soll verstanden werden, dass auch im Stand der Technik bekannten Vorrichtungen während des normalen Betriebs immer wieder Konzentrat aus der Anlage herausgeleitet werden kann. Die vorliegende Erfindung erlaubt jedoch vorteilhaft, das verworfene Volumen abhängig von dem durch die Güteparametersensor detektierten Gütemaß einzustellen. Mit anderen Worten, wenn die Leitfähigkeit über einen Grenzwert steigt, kann das Konzentratvolumen, welches über das Ventil verworfen wird, ggf. erhöht werden, um die Leitfähigkeit des Prozesswassers zu regulieren, insbesondere zu senken, und um somit eine bessere Voraussetzung für Permeat mit niedrigerer Leitfähigkeit zu schaffen.

Erfindungsgemäß sind der erste Güteparametersensor und der wenigstens eine weitere Güteparametersensor bevorzugt fluidisch nach der Membran angeordnet. Das heißt, dass der erste Güteparameter der ersten Umkehrosmose-Prozessflüssigkeit nach Durchlaufen der wenigstens einen Membran detektiert wird.

Die oben beschriebenen Aufgaben werden ebenfalls erfindungsgemäß gelöst durch ein erfindungsgemäßes Verfahren zur Überwachung von Güteparametern von Umkehrosmose-Prozessflüssigkeiten. Dieses Verfahren dient bevorzugt der Überwachung sicherheitsrelevanter Parameter im erzeugten Permeat einer Umkehrosmoseanlage. Die Umkehrosmoseanlage ist dabei bevorzugt eine Umkehrosmoseanlage nach der vorliegenden Erfindung bzw. umfass bevorzugt eine Vorrichtung nach der vorliegenden Erfindung. Dabei wird durch redundante Erfassung und Verarbeitung von Sensordaten durch zwei Prozessoren die Betriebssicherheit der Anlage vorteilhaft erhöht. Das erfindungsgemäße Verfahren umfasst dabei wenigstens die folgenden Schritte:

einen Schritt a) des Detektierens eines ersten Sensorwertes eines ersten Güteparameters eines Permeats der Umkehrosmose-Prozessflüssigkeiten mittels eines ersten Güteparametersensors und ein Weiterleiten des ersten Sensorwertes an einen ersten Prozessor und einen Schritt b) des Detektierens eines zweiten Sensorwertes des ersten Güteparameters des Permeats der Umkehrosmose-Prozessflüssigkeiten mittels eines weiteren Güteparametersensors und ein Weiterleiten des zweiten Sensorwertes an einen zweiten Prozessor. Es soll verstanden werden, dass die beiden Schritte des Detektierens des ersten und des zweiten Sensorwertes unabhängig voneinander erfolgen können. Dabei wird bevorzugt jeder Detektionsschritt wenigstens einmal zu einem vorbestimmten Zeitpunkt ausgeführt und/oder in einem vorbestimmten Zeitintervall mehrfach wiederholt. Damit kann durch eine vorbestimmte, ggf. mehrfache und/oder regelmäßige Detektion der Sensorwerte des ersten Güteparameters des Permeats, die Güte des Permeats insbesondere über längere Zeiträume und/oder zu ausgewählten Zeitpunkten überwacht werden. In einem Schritt c) findet der Datentransfers zwischen dem ersten Prozessor und dem zweiten Prozessor mittels der Kommunikationsschnittstelle statt. Dabei werden insbesondere die Sensordaten, roh oder in durch die Prozessoren prozessierte Form von einem zum anderen Prozessor übergeben. Dieser Austausch kann unidirektional und/oder bidirektional zwischen den Prozessoren erfolgen. Insbesondere kann ein erster Prozessor als Hauptprozessor und ein zweiter Prozessor als Nebenprozessor dienen. Beispielsweise sendet der Nebenprozessor den erfassten/berechneten Leitfähigkeitswert an den Hauptprozessor und/oder umgekehrt. Der Datentransfer findet dabei über die Kommunikationsschnittstelle statt.

In einem Schritt d) des erfindungsgemäßen Verfahrens wird, um insbesondere festzustellen, ob eine Abweichung des ersten Sensorwertes von dem zweiten Sensorwert vorliegt, die das Auslösen einer Regelmaßnahme erfordern würde, eine Abweichung des ersten Sensorwertes von dem zweiten Sensorwert mittels des ersten Prozessors und/oder des zweiten Prozessors berechnet. Damit kann insbesondere festgestellt werden, wie nahe die beiden Messwerte beieinander liegen. Da mit den beiden unabhängigen Prozessoren der Güteparameter zweimal unabhängig voneinander detektiert wird, ist eine Abweichung insbesondere indikativ für eine mögliche Fehlfunktion eines der Sensoren und/oder eines der Prozessoren und/oder einer anderen Funktion der Umkehrosmoseanlage.

Um insbesondere festzustellen, ob einer oder beide der detektierten Sensorwerte in einem Bereich liegen, der das Auslösen einer Regelmaßnahme erfordern würde, wird in einem Schritt f) der erste Sensorwert und/oder der zweite Sensorwert des ersten Güteparameters eines Permeats der Umkehrosmose-Prozessflüssigkeiten mit einem vorbestimmten maximalen Sensorgrenzwert verglichen. Dabei ist der vorbestimmten maximale Sensorgrenzwert bevorzugt so gewählt, dass ein Dauerhaftes Über- bzw. Unterschreiten dieses Wertes bezogen auf den jeweiligen Güteparameter zu vermeiden wäre. Mit anderen Worten wird also neben dem Vergleichen der Abweichung der Güteparameter, also zum Beispiel der Leitfähigkeiten, untereinander, der gemessene und ggf. berechnete Güteparameter, also zum Beispiel die gemessene/berechnete Leitfähigkeit, auch gegen einen absoluten Wert verglichen.

Das erfindungsgemäße Verfahren hat dabei Vorteile gegenüber dem Stand der Technik. Insbesondere können die Prozessoren jeweils überprüft werden, ohne, dass der Betrieb der Anlage unterbrochen werden müsste. Sollte der Hauptprozessor bspw. aus irgendeinem Grund nicht mehr funktionieren, zum Beispiel durch Endlosschleifen, zu viele empfangene Anfragen, zu viele gesendete Anfragen, Thread eingefroren, Bauteildefekte, oder ähnliches, kann dies vom Nebenprozessor dadurch erkannt werden, dass der Hauptprozessor keinen Sensorwert mehr über die Kommunikationsschnittstelle 117 sendet und/oder anfordert. Gleichsam kann der Nebenprozessor 116 auf Funktionalität geprüft werden.

Weiterhin erlaubt das erfindungsgemäße Verfahren eine einfache Detektion von Sensordefekten. Da Leitfähigkeit, in der Regel direkt über einen Strom gemessen wird, ist eine klassische Unterscheidung zwischen einer sehr kleinen Leitfähigkeit und einem Sensordefekt, z.B. Drahtbruch, schwierig. Durch das unabhängige Messprinzip kann ein Sensordefekt identifiziert werden.

Schließlich erlaubt die vorliegende Erfindung der Umkehrosmoseanlage eine Kommunikation von Betriebszuständen, beispielsweise als Regelmaßnahme.

Durch die Kommunikationsschnittstelle kann die Betriebsphase gesendet werden und die Leitfähigkeitsüberwachung somit Abhängigkeit des Betriebsmodus an und ausschalten. Auch kann bei einem auftretenden Fehler die Anlage neu gestartet werden, um beispielsweise ionenhaltiges Permeat an den Leitfähigkeitssonden abzutransportieren.

Es sei im Zusammenhang mit dem erfindungsgemäßen Verfahren auch darauf hingewiesen, dass die angegebenen Schritte nicht zwangsläufig in der angegebenen Reihenfolge durchgeführt werden müssen. Die angegebenen Schritte können in jeder anderen geeigneten Reihenfolge oder auch gleichzeitig durchgeführt werden.

Allerdings kann die oben angegebene Reihenfolge für bestimmte Ausführungsformen des erfindungsgemäßen Verfahrens vorteilhaft sein. Gleichzeitig kann zum Beispiel ein Schritt a) des Detektierens eines ersten Sensorwertes vor einem Schritt b) des Detektierens eines zweiten Sensorwertes oder sogar gleichzeitig durchgeführt werden.

Es wird zudem von einem Fachmann anerkannt werden, dass ein Merkmal, eine Ausführungsform, eine Wirkung oder ein Vorteil, wie hier in Verbindung mit der erfinderischen Vorrichtung und/oder der erfinderischen Umkehrosmoseanlage beschrieben, auch ein Merkmal, eine Ausführungsform, eine Wirkung oder ein Vorteil des erfinderischen Verfahrens sein kann, bzw. umgekehrt.

**In** einer vorteilhaften Ausführungsform der erfindungsgemäßen Vorrichtung ist die Steuerungseinheit dazu eingerichtet, abhängig von der Abweichung des ersten Sensorwertes von dem zweiten Sensorwert die Umkehrosmoseanlage, insbesondere die wenigstens eine erste Pumpe oder die wenigstens eine weitere Pumpe, abzuschalten, so dass die Zuführung von Permeat an wenigstens ein medizinisches Endgerät, insbesondere Dialysegerät, unterbunden wird. Zusätzlich oder alternativ kann dabei auch ein Vergleichen des ersten Sensorwertes und/oder des zweiten Sensorwerts mit einem vorbestimmbaren Grenzwert des ersten Güteparameters zum Abschalten als physikalische Regelmaßnahme führen.

In einer weiteren vorteilhaften Ausführungsform der erfindungsgemäßen Vorrichtung ist die Steuerungseinheit eingerichtet, abhängig von der Abweichung des ersten Sensorwertes von dem zweiten Sensorwert und/oder dem Vergleichen des ersten Sensorwertes und/oder des zweiten Sensorwerts mit einem vorbestimmbaren Grenzwert des ersten Güteparameter des Permeats der ersten Umkehrosmose-Prozessflüssigkeit, die Pumpenleistung der wenigstens einen ersten Pumpe und/oder der wenigstens einen weiteren Pumpe für einen vorbestimmten Zeitraum zu erhöhen. Dies erlaubt vorteilhaft, dass der am ersten Güteparametersensor und/oder am wenigstens einen weiteren Güteparametersensor gemessene Sensorwert des ersten Güteparameters des Permeats vorteilhaft reduziert wird.

Es soll verstanden werden, dass ein Abschalten als physikalische Regelmaßnahme eine Sicherheitsmaßnahme darstellt, jedoch den Prozess, die Versorgung der medizinischen Endgeräte, und damit der Patienten, mit Permeat, verhindert. Dies kann insbesondere vorteilhaft sein, wenn anhand des Verhaltens der Güteparameter ein Schaden für die Patienten zu befürchten ist. Wenn bspw. ein extremer Ausschlag des Güteparameters eine Permeatgüte vermuten lässt, die unmittelbar schädlich für die Einleitung des Permeats in das wenigstens eine medizinische Endgerät und/oder den Patienten wäre, oder ein deutlich überhöhter Gütewert über einen längeren Zeitraum gemessen werden würde. Es wird verstanden werden, dass mildere Regelmaßnahmen bevorzugt sind, die zunächst den Betrieb des wenigstens einen medizinischen Endgerätes aufrechterhalten können. Dabei ist es besonders bevorzugt, die Güte des Permeats mittel der erfindungsgemäßen Vorrichtung und/oder des erfindungsgemäßen Verfahrens so zu beeinflussen, dass diese wieder in die vorbestimmten Grenzwerte zurückgeführt wird.

In einer weiteren vorteilhaften Ausführungsform des erfindungsgemäßen Verfahrens nach der vorliegenden Erfindung kann in einem Schritt des Vornehmens einer Regelmaßnahme basierend auf dem Ergebnis des Vergleichens in Schritt e) und/oder Schritt f) die Steuereinheit die Regelmaßnahme vornehmen oder ansteuern. Die Regelmaßnahme kann dabei das Erzeugen einer Statusmeldung umfassen, insbesondere einer Statusmeldung über den störungsfreien Betrieb der Umkehrosmoseanlage oder das Auftreten einer Störung und/oder das Abweichen eines Sensorwertes. Zusätzlich oder alternativ, kann die Steuereinheit auch eine physikalische Maßnahme als Regelmaßnahme vornehmen oder ansteuern.

Eine solche physikalischen Maßnahme kann dabei in einer Regelung der Pumpenleistung einer Pumpe, bevorzugt der ersten Pumpe, insbesondere Druckpumpe bestehen und/oder in einer Regelung der Pumpenleistung einer weiteren Pumpe, insbesondere Zirkulationspumpe. Zusätzlich oder alternativ kann auch ein Ventil, insbesondere ein Ablassventil für Konzentrat aus dem System, geregelt oder eine andere geeignete Regelmaßnahme durchgeführt werden.

In einer bevorzugten Ausführungsform des erfindungsgemäßen Verfahrens, umfasst das Verfahren einen Schritt des Erhöhens der Pumpenleistung der wenigstens einen ersten Pumpe und/oder der wenigstens einen Pumpe für einen vorbestimmten Zeitraum. Dabei ist es bevorzugt, dass das Erhöhen abhängig ist von der in dem Schritt d) berechneten Abweichung des ersten Sensorwertes von dem zweiten Sensorwerts. Gleichzeitig oder alternativ ist das Erhöhen bevorzugterweise abhängig von dem Vergleichen in dem Schritt f) des ersten Sensorwertes und/oder des zweiten Sensorwertes des ersten Güteparameters eines Permeats der Umkehrosmose-Prozessflüssigkeiten mit einem vorbestimmten Sensorgrenzwert.

In einer weiteren vorteilhaften Ausführungsform der erfindungsgemäßen Vorrichtung nach der vorliegenden Erfindung, umfasst die Vorrichtung ein erstes printed circuit board (PCB) auf dem der erste Prozessor und der zweite Prozessor, bevorzugt mittels galvanischer Trennung, zwischen dem ersten und dem zweiten Prozessor angeordnet sind. Zusätzlich oder alternativ können Prozessoren, insbesondere der erste Prozessor und der zweite Prozessor auf zwei separierten printed circuit boards (PCB) angeordnet sein.

In einer weiteren vorteilhaften Ausführungsform der erfindungsgemäßen Vorrichtung nach der vorliegenden Erfindung, ist die Kommunikationsschnittstelle zur Kommunikation mit einer Überwachungseinheit eingerichtet, wobei die Überwachungseinheit außerhalb der Umkehrosmoseanlage angeordnet ist. Dies kann es ermöglichen, eine Überwachungseinheit außerhalb der Vorrichtung und insbesondere außerhalb der Umkehrosmoseanlage bereitzustellen. Beispielsweise kann die Kommunikationsschnittstelle zur drahtlosen Kommunikation, insbesondere über eine Netzwerkverbindung, Bluetooth, oder ähnliches mit einer Überwachungseinheit kommunizieren. Hierfür ist es vorteilhaft, dass die Kommunikationsschnittstelle einen Datenaustausch zwischen der Überwachungseinheit und den Prozessoren ermöglicht. Die Überwachungseinheit kann insbesondere teil eines Computers, eines Computerprogramms, insbesondere einer App sein.

In einer vorteilhaften Ausführungsform der erfindungsgemäßen Umkehrosmoseanlage nach der vorliegenden Erfindung, umfasst die Umkehrosmoseanlage wenigstens einen weiteren Güteparametersensor zur Erfassung einer Leitfähigkeit und/oder eines weiteren Güteparameters der Umkehrosmose-Prozessflüssigkeiten, insbesondere des Permeats. Dabei ist wiederum die Steuereinheit und/oder einer der vorhandenen oder ein weiterer Prozessor zur Erfassung und Verarbeitung der entsprechenden Sensordaten eingerichtet. Der wenigstens eine weitere Güteparameter der Umkehrosmose-Prozessflüssigkeiten ist bevorzugt ausgewählt ist aus der Gruppe umfassend Leitfähigkeit, Temperatur, Volumenstrom, Druck. Insbesondere kann in einer Ausführungsform der vorliegenden Erfindung der Rückhalt berechnet werden. Der Rückhalt ist dem Fachmann bevorzugt als der Wert bekannt, welcher angibt, wie viele Ionen aus dem Wasser herausgefiltert werden. Im Rahmen der vorliegenden Erfindung kann bevorzugt eine Weichwasserleitfähigkeit oder Prozesswasserleitfähigkeit (insb. vor der Membran) gemessen werden, um den Rückhalt zu berechnen, wobei ein Gütesensor zur Detektion der Weichwasserleitfähigkeit vorteilhaft am Rücklauf angeordnet ist.

Die Berechnung des Rückhalts kann dabei insbesondere über die nachfolgende Formel erfolgen:

$$\text{Formel I: } Rückhalt\ [\%] = (1 - (\text{LFPermeat}/\text{LFProzesseingangswasser})) * 100,$$

wobei LF Permeat für die Leitfähigkeit des Permeats und LF Prozesseingangswasser für die Leitfähigkeit des Prozesseingangswassers steht.

Ein Güteparametersensor zur Erfassung einer Leitfähigkeit von Prozesseingangswasser kann insbesondere an der Zuleitung von Prozesseingangswasser und/oder im Zuleitungsbereich eines Vorlagetankes angeordnet werden.

Die Leitfähigkeit des Prozesseingangswassers wird in bevorzugter Weise ebenfalls individuell von beiden Prozessoren gemessen. Alternativ kann die Leitfähigkeit auch nur von einem Prozessor gemessen werden und über die Kommunikationsschnittstelle ausgetauscht werden. Dies ist möglich, da die Leitfähigkeit im Prozesseingangswasser keinen größeren Schwankungen unterliegt.

In einer weiteren vorteilhaften Ausführungsform der erfindungsgemäßen Umkehrosmoseanlage nach der vorliegenden Erfindung, ist der wenigstens einen weiteren Güteparametersensor zur Erfassung einer Leitfähigkeit und/oder eines weiteren Güteparameters zwischen der wenigstens einen, bevorzugt Druck-, Pumpe und/oder der wenigstens einen weiteren, bevorzugt Zirkulations-, Pumpe und der wenigstens einen Membran angeordnet. Dies erlaubt vorteilhaft die Detektion eines Sensorwertes eines Güteparameters der Umkehrosmose-Prozessflüssigkeit vor dem Kontaktieren mit der Membran und ggf. nach einer Beimischung von Konzentrat.

In einer weiteren vorteilhaften Ausführungsform der erfindungsgemäßen Umkehrosmoseanlage nach der vorliegenden Erfindung, ist der wenigstens eine weitere Güteparametersensor zur Erfassung einer Leitfähigkeit und/oder eines weiteren Güteparameters zur Messung des Güteparameters im Prozesseingangswasser angeordnet.

In einer weiteren vorteilhaften Ausführungsform der erfindungsgemäßen Umkehrosmoseanlage nach der vorliegenden Erfindung ist wenigstens eine weitere Filter-Pumpen-Einheit vorgesehen, die wenigstens eine weitere Pumpe und wenigstens eine weitere Membran umfasst, wobei die wenigstens eine weitere Filter-Pumpen-Einheit in Reihe, mit der wenigstens einen ersten Filter-Pumpen-Einheit fluidisch verbunden ist. Mit anderen Worten ist nach einer ersten Membran eine weitere Pumpe und eine weitere Membran angeordnet, und der ersten Membran nachgeschaltet.

Es soll auch verstanden werden, dass wie hierin verwendet der Begriff "Membran" bevorzugt eine funktionelle Einheit bezeichnet, die eine einzelne Membran oder eine Mehrzahl von Membranen umfasst.

In einer weiteren vorteilhaften Ausführungsform des erfindungsgemäßen Verfahrens nach der vorliegenden Erfindung, werden Sensorwerte wenigstens eines weiteren Güteparameters der Umkehrosmose-Prozessflüssigkeiten, insbesondere des Permeats, mittels eines entsprechenden Güteparametersensors detektiert und in Schritten c) bis f) verwendet. Mit anderen Worten wird neben einem ersten Güteparameter, bspw. der Leitfähigkeit des Permeats, wenigstens ein weiteren Güteparameter der Umkehrosmose-Prozessflüssigkeiten, zum Beispiel eine Temperatur des Permeats, oder die Leitfähigkeit des Weichwassers detektiert, bzw. in dem erfindungsgemäßen Verfahren verwendet. Dabei ist der wenigstens eine weitere Güteparameter der Umkehrosmose-Prozessflüssigkeiten bevorzugt ausgewählt aus der Gruppe umfassend Leitfähigkeit, Temperatur, Volumenstrom, Druck.

Insbesondere kann die Temperatur der Umkehrosmose-Prozessflüssigkeiten, insbesondere des Permeats im erfindungsgemäßen Verfahren mit einem entsprechenden Temperatursensor detektiert und als Güteparameter verwendet werden. Insbesondere kann die Temperatur als zusätzlicher Güteparameter zur Leitfähigkeit verwendet werden. Damit erlaubt das erfindungsgemäße Verfahren vorteilhaft auch die Verwendung einer Temperatur-kompensierten Leitfähigkeit bzw. die Temperatur kann ebenfalls nach dem vorangegangen beschriebenen Verfahren als unabhängiger Güteparameter zusätzlich überwacht werden.

In einer weiteren vorteilhaften Ausführungsform des erfindungsgemäßen Verfahrens nach der vorliegenden Erfindung wird der vorbestimmte Abweichungsgrenzwert in Schritt e) abhängig von dem ersten Sensorwert und/oder dem zweiten Sensorwert vorbestimmt. Dies erlaubt insbesondere die Verwirklichung und Verwendung von dynamischen Grenzwerten im erfindungsgemäßen Verfahren. Insbesondere in Phasen mit relativ niedriger Leitfähigkeit sorgen kleine Abweichungen zwischen den beiden Messungen zu großen prozentualen Abweichungen. Um dies zu kompensieren, kann der Schwellwert der prozentualen Leitfähigkeit an die gemessene Leitfähigkeit gekoppelt werden. Dies kann bspw. entweder über eine Gleichung (z.B. erster Ordnung) oder einen Look-Up Table durchgeführt werden.

Die oben beschriebenen Aufgaben werden ebenfalls erfindungsgemäß gelöst durch ein Computerprogramm und/oder ein das Computerprogramm umfassendes Speichermedium, wobei das Computerprogramm Anweisungen aufweist, die bei Ausführung durch einen Computer diesen veranlassen, das Verfahren nach der vorliegenden Erfindung auszuführen.

Alle beschriebenen Ausführungsformen der Erfindung haben den Vorteil, dass bei Ausfall oder Störung der Sensorik oder einem Softwarefehler der Steuerungseinheit eine Ausfallkompensation und insbesondere eine Redundanz in der Pumpenabschaltung gegeben ist. Damit wird die Betriebssicherheit der Umkehrosmoseanlage erhöht. Dadurch entsteht vorteilhafterweise die Möglichkeit, die Anlage zu stoppen, auch wenn ein Sensor und/oder ein Prozessor ausfallen würde oder beeinträchtigt wäre. Mit der erfindungsgemäßen Vorrichtung kann dann eine fortwährende Zustandskontrolle erreicht werden und ggf. Maßnahmen durchgeführt werden, um den Betrieb der Umkehrosmoseanlage aufrecht zu erhalten. Vorteilhaft wird weiterhin das Auftreten von falsch positiven Alarmen bei chemischer und thermischer Desinfektion vermieden werden, obwohl die Leitfähigkeiten des Permeats bei diesen Betriebsphasen über die erlaubten Grenzwerte steigen können und somit eine Abschaltung ungewollt ist. Weiterhin kann vorteilhaft der erste Prozessor und der zweite Prozessor leicht auf Funktionalität geprüft werden, ohne die erfindungsgemäße Umkehrosmoseanlage abschalten zu müssen.

## Zusammenfassende Beschreibung der Erfindung

Es ist deshalb eine der vorliegenden Erfindung zu Grunde liegende Aufgabe die Nachteile von Umkehrosmoseanlagen im Stand der Technik zu überwinden. Insbesondere ist es eine der vorliegenden Erfindung zu Grunde liegende Aufgabe, Umkehrosmoseanlagen bereitzustellen, die über eine verbesserte Ausfallkompensation, Sicherheit und/oder eine verbesserte Zustandskontrolle verfügen.

## Kurzbeschreibung der Figuren

Die vorliegende Erfindung wird im Folgenden ausführlicher erläutert unter Bezugnahme auf die Zeichnungen, aus denen weitere Merkmale, Ausführungsformen und Vorteile entnommen werden können. In den in den Abbildungen gezeigten Ausführungsformen werden Elemente, die ähnliche oder identische Funktionen haben, mit gleichen Bezugszeichen versehen. Es wird darauf hingewiesen, dass die Abbildungen möglicherweise nicht maßstabsgetreu zueinander sind.

Dabei zeigt:

FIG 1 eine Vorrichtung nach dem Stand der Technik mit einer Sensorik und einem Prozessor;

FIG 2 zeigt eine weitere Vorrichtung nach dem Stand der Technik mit zwei unabhängigen Sensoriken und unabhängigen Prozessoren;

FIG 3A und 3B zeigen Ausführungsformen der vorliegenden Erfindung;

FIG 4 zeigt einen schematischen Ablauf der Prozess-Schritte des erfindungsgemäßen Verfahrens in einer ersten Ausführungsform; und

FIG 5 zeigt eine Messung der Leitfähigkeit als Güteparameter in einer weiteren Ausführungsform des erfindungsgemäßen Verfahrens nach der vorliegenden Erfindung.

## Detaillierte Beschreibung

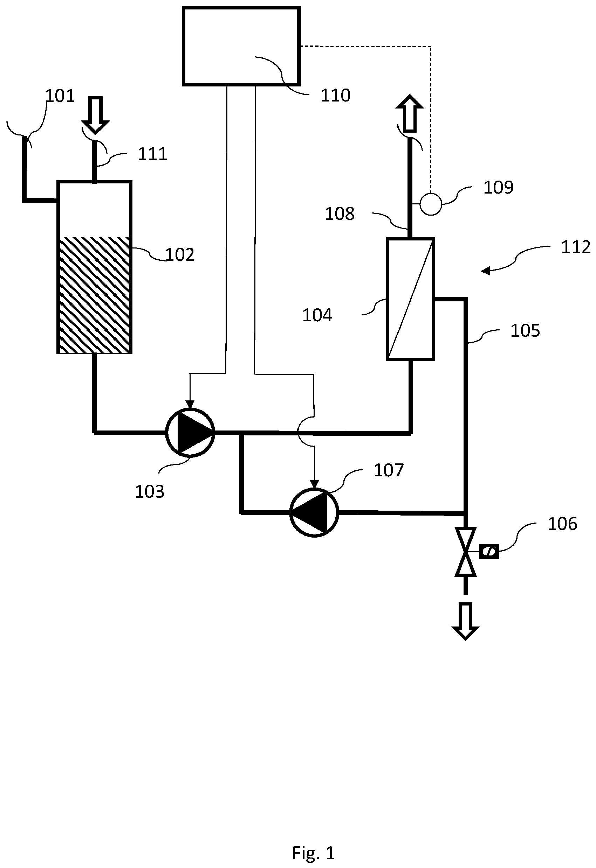

FIG 1 zeigt eine schematische Darstellung einer Umkehrosmoseanlage nach dem Stand der Technik. Diese Anlagen sind in der Regel so aufgebaut, dass das Prozesseingangswasser 101 in einen Vorlagetank 102 geleitet wird. Anschließend saugt eine Druckpumpe 103 das benötigte Wasser aus dem Vorlagetank 102 an und presst dieses in die Membranen 104. Aus diesem Prozess entsteht Permeat 108 als Produkt und Konzentrat 105 als Nebenprodukt. Um Wasser zu sparen kann ein Teil des Konzentrats 105 über eine Zirkulationspumpe 107 in den Prozess wiedereingespeist werden. Um einen Teil der zurückgehaltenen Ionen aus der Anlage zu befördern, wird das Magnetventil 106 geöffnet, um Konzentrat 105 in das Abwasser zu befördern.

Das Permeat 108 wird in eine Ringleitung eingespeist, wo dieses von einem oder mehreren medizinischen Endgeräten, zum Beispiel Dialysegeräten, abgenommen werden kann. Nicht verbrauchtes Permeat 108 gelangt über den Rücklauf 111 zurück in den Vorlagetank 102. In der Regel wird zur Überwachung des Filterprozesses die Leitfähigkeit des Permeats 108 als Güteparameter des Permeats 108 der Osmose-Prozessflüssigkeit, durch einen ersten Güteparametersensor 109, üblicherweise ein Leitfähigkeitssensor 109 detektiert. Die Messung und ggf. Verarbeitung der Sensorwerte werden über eine Steuerungseinheit 110, welche innerhalb der Umkehrosmoseanlage 112 ist, durchgeführt. Sollte die Steuerungseinheit 110 über den Leitfähigkeitssensor 109 einen Anstieg über einen festgelegten Grenzwert registrieren, wird die Umkehrosmoseanlage 112 gestoppt. Der Stoppvorgang wird dadurch realisiert, dass die Druckpumpe 103 und die Zirkulationspumpe 107 über die Steuerungseinheit 110 gestoppt wird. Es ist dabei offensichtlich, dass bei einer Fehlfunktion des Sensors 109 oder der Steuerungseinheit 110 das Stoppen der Pumpen die einzige vorgesehene Regelmaßnahme ist. Damit führt eine einfache Fehlfunktion, ein Softwarefehler, oder ähnliches zu einem unmittelbaren Stoppen der Pumpen, auch wenn die tatsächliche Leitfähigkeit des Permeats 108 nicht angestiegen ist. Andererseits käme es nicht zu der Abschaltung, und die Steuerungseinheit 110 würde einen entsprechenden Software -Fehler aufweisen, oder der Leitfähigkeitssensor 109 einen Defekt haben, könnte ebenfalls ein Problem in der Pumpenabschaltung oder Sonstiges erfolgen und es gäbe keine Möglichkeit die Anlage zu stoppen.

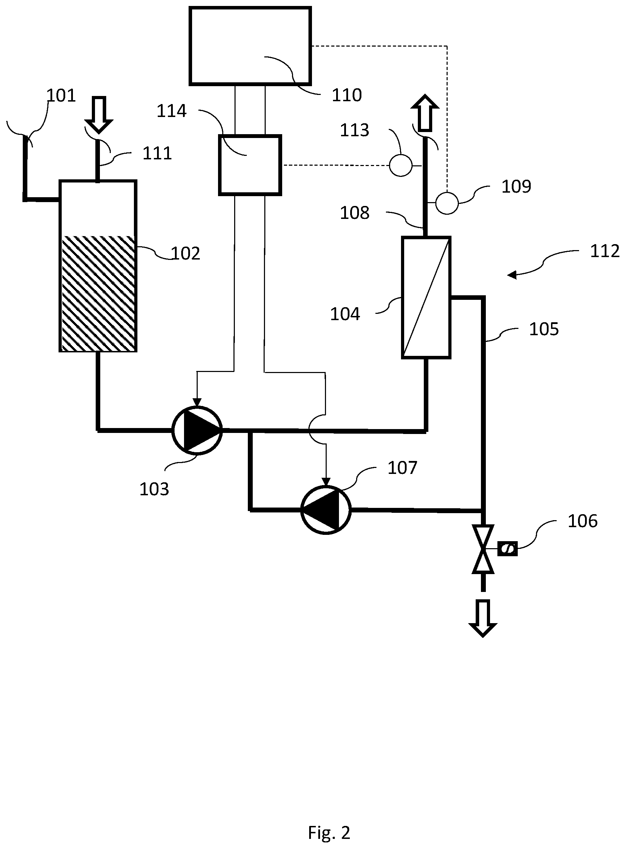

In FIG 2 ist eine schematische Abbildung einer im Stand der Technik bekannten Weiterbildung der in FIG 1 gezeigten Umkehrosmoseanlage dargestellt. Dabei ist ein weiteres von der primären Steuerungseinheit 110 unabhängiges Messgerät in die Umkehrosmoseanlage 112 eingebaut, welches ebenfalls mit einem Leitfähigkeitssensor 113 die Qualität des Permeats 108 überwacht. Die zweite Steuerungseinheit 114 weist einen zweiten Prozessor auf, der zur Erfassung und Verarbeitung eines durch wenigstens einen weiteren Güteparametersensor 113 detektierten zweiten Sensorwert der Leitfähigkeit als erstem Güteparameter des Permeats 108 eingerichtet ist. Sollte diese sekundäre Steuerungseinheit 114 einen Anstieg der Leitfähigkeit über einen erlaubten Grenzwert feststellen, wird die Umkehrosmoseanlage 112 gestoppt wie vorrangegangen im Zusammenhang mit FIG 1 beschrieben. Dabei besteht das Ziel der sekundären Steuerungseinheit 114 darin, ein potenzielles Versagen der primären Steuerungseinheit 110 zu kompensieren und somit eine Ausfallkompensation gegenüber der in FIG 1 gezeigten Anlage mit nur einer Sensorik und nur einer Steuerungseinheit zu erreichen.

Nachteilig am in FIG 2 gezeigten Stand der Technik mit zweiter Steuerungseinheit 114 ist jedoch die fehlende Zustandskontrolle und die fehlende Möglichkeit zur Rücksetzungsmaßnahme. Mit anderen Worten besitzt die zweite Steuerungseinheit 114 keine Informationen über den aktuellen Betriebs-Modus und insbesondere keine Informationen über die Funktion der ersten Steuerungseinheit 110 und des ersten Sensors 109. Dadurch kann es zu falsch positiven Alarmen, insbesondere bei chemischer und thermischer Desinfektion kommen. Die Leitfähigkeiten können bei diesen Betriebsphasen über die erlaubten Grenzwerte steigen und somit zu einer ungewollten Abschaltung führen. Ebenfalls kann durch längere Standzeit ein falsch positiver Fehler entstehen. Durch den drucklosen Zustand an der Membran beginnen Ionen in das Permeat 108 zu diffundieren. Dadurch kommt es zu einem Anstieg der Leitfähigkeit im Permeat 108 abhängig vom osmotischen Druck und der Zeit. Sobald der Grenzwert der sekundären Steuerungseinheit 114 überschritten wurde, kann die Anlagen nicht mehr gestartet werden. Dies führt dazu, dass der Fehler vom Betreiber nicht behoben werden kann und eine technisch geschulte Person die Anlage unter größeren Eingriffen wieder in Betrieb nehmen muss. Während diesen Zeiten ist ein Betrieb der medizinischen Endgeräte, und insbesondere keine Behandlungen im Dialysezentrum, möglich. Zudem ist die in FIG 2 gezeigte Umsetzung des Standes der Technik in der Praxis meist kostenintensiv. Üblicherweise wird im Stand der Technik die Software beim Fehlerauftreten in einen Aus-Modus versetzt und der Nutzer wird über das Problem benachrichtigt. Der Nutzer hat dann ggf. die Möglichkeit, einen, bevorzugt zeitlich eingeschränkten (~1 min ) Spülmodus durchzuführen oder alternativ einen Dialysemodus erneut zu starten, um den Fehler zu verifizieren. Sollte die Membran intakt sein kann im Stand der Technik durch diese Maßnahmen das Permeat mit hoher Leitfähigkeit weggespült werden und die Anlage kann wie gewohnt betrieben werden. Ist die Membran defekt, bleibt die Leitfähigkeit hoch und der Fehler bleibt weiterhin aktiv. Üblicherweise wird im Stand der Technik die Leitfähigkeit in diesem Betrieb nicht überwacht, da keine Patienten währen diesen Betriebsphasen mit der Vorrichtung behandelt werden. Deshalb ist der Datenaustausch auch relevant (Austausch von Betriebszuständen). Ganz generell steigt die Leitfähigkeit während diesen Betriebsphasen stark an.

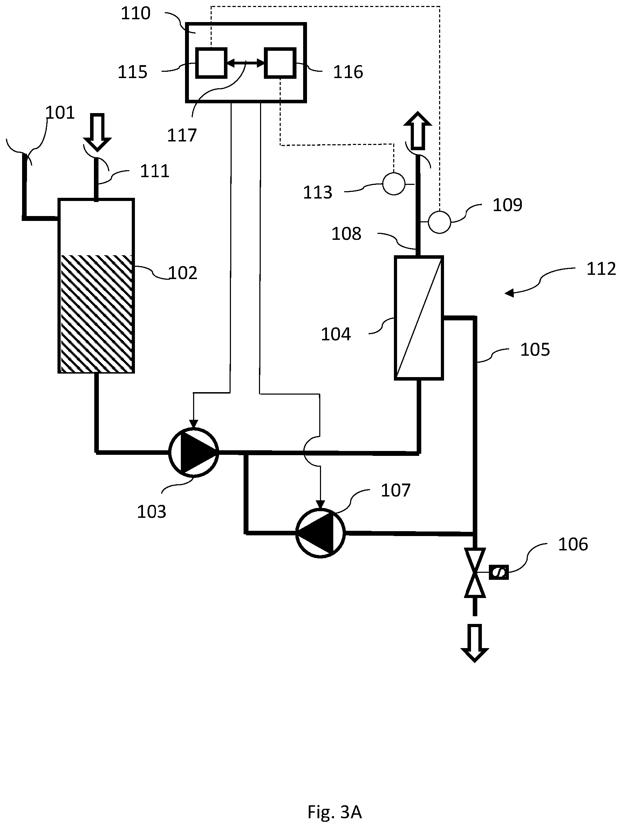

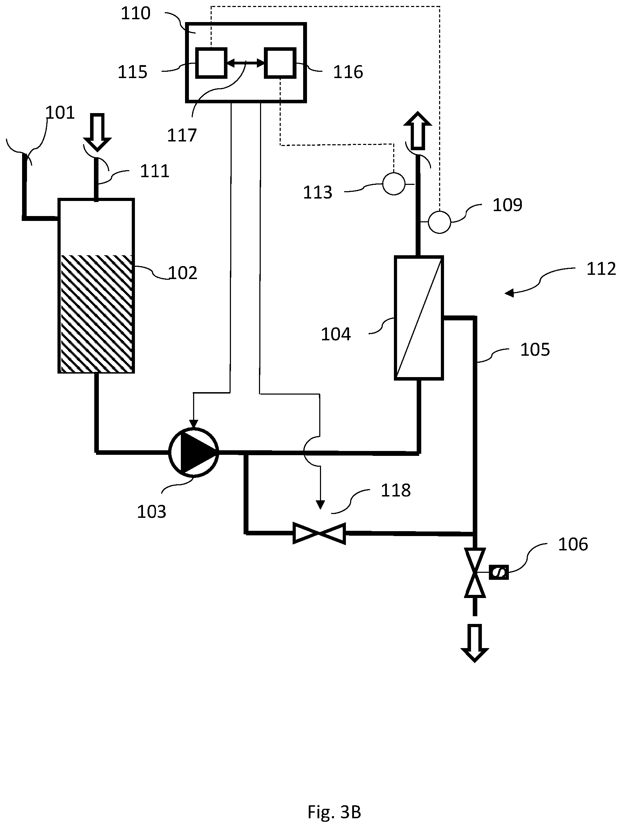

In FIG 3 ist eine Umkehrosmoseanlage 112 gemäß einer Ausführungsform der vorliegenden Erfindung gezeigt. Dabei ist ein Vorlagetank 102 vorgesehen, der über eine Prozesseingangswasserzuführung 101 mit Prozesswasser versorgbar ist und weiter eine Rücklauf-Zuführung 111 umfasst, mittels der Rücklaufflüssigkeit aus einer mit dem wenigstens einen medizinischen Endgerät, insbesondere Dialysegerät, verbundenen Ringleitung in den Vorlagetank 102 einspeisbar ist (nach unten zeigender Pfeil links in der Abbildung). Ebenfalls ist eine Filter-Pumpen-Einheit, mit einer Druckpumpe 103 und einer Zirkulationspumpe 107 vorgesehen, sowie eine Membraneinheit mit einer oder mehreren Membranen 104, die fluidisch mit dem Vorlagetank 102 verbunden sind und zur Erzeugung des Permeats 108 aus der Umkehrosmose-Prozessflüssigkeiten des Vorlagetanks 102 dienen. Die Steuereinheit 110 ist eingerichtet die Pumpenleistung der ersten Pumpe 103 und der zweiten Pumpe 107 voneinander unabhängig zu steuern. Wie im Stand der Technik in FIG 1 und FIG 2 ist dabei die erste Pumpe 103 so angeordnet, dass die Membran 104 mittels der Pumpe 103 mit Umkehrosmose-Prozessflüssigkeit aus dem Vorlagetank 102 zur Erzeugung des Permeats 108 beaufschlagt werden kann, und das Permeat 108 sodann mittels der Permeatzuleitung (nach oben zeigender Pfeil rechts in der Abbildung) über die Ringleitung dem wenigstens einen medizinischen Endgerät, insbesondere Dialysegerät, zuführbar ist. Ein erster Güteparametersensor 109 zum Detektieren eines ersten Sensorwertes eines ersten Güteparameters des Permeats 108 der Umkehrosmose-Prozessflüssigkeiten, bevorzugt die Leitfähigkeit des Permeats, und wenigstens ein weiterer Güteparametersensor 113 zum Detektieren eines zweiten Sensorwertes des ersten Güteparameters des Permeats 108 der Umkehrosmose-Prozessflüssigkeiten detektieren den Güteparameter. Die Umkehrosmoseanlage in FIG 3A bzw. FIG 3B umfasst weiter eine Vorrichtung zur Überwachung der Umkehrosmoseanlage 112 nach der vorliegenden Erfindung. Im Unterschied zum Stand der Technik fehlt also eine zweite Steuerungseinheit 114. Dabei ist in der Vorrichtung nach der vorliegenden Erfindung eine Steuerungseinheit 110 vorgesehen, mit wenigstens einem ersten Prozessor 115 und einem zweiten Prozessor 116, sowie einer Kommunikationsschnittstelle 117, die zum Datentransfer zwischen dem ersten Prozessor 115 und dem zweiten Prozessor 116 eingerichtet ist. Der erste Prozessor 115 erfasst und verarbeitet ein durch einen ersten Güteparametersensor 109 detektierten ersten Sensorwertes eines ersten Güteparameters eines Permeats 108 der Umkehrosmose-Prozessflüssigkeiten, beispielsweise der Leitfähigkeit. Gleichsam erfasst und verarbeitet unabhängig davon der zweite Prozessor 116 einen durch wenigstens einen weiteren Güteparametersensor 113 detektierten zweiten Sensorwertes des ersten Güteparameters des Permeats 108. Die Steuerungseinheit 110 berechnet eine Abweichung des ersten Sensorwertes von dem zweiten Sensorwert und vergleicht beide Sensorwerte unabhängig voneinander mit einem vorbestimmbaren Grenzwert des ersten Güteparameters. Es wird also zum einen festgestellt, ob die Sensorwerte im Wesentlichen übereinstimmen oder voneinander abweichen. Zum anderen wird festgestellt, ob die Sensorwerte innerhalb der vorbestimmbaren Grenzwerte liegen. Dabei könne Grenzwerte sinnvoll abhängig von dem gewählten Parameter gesetzt werden. Beispielsweise macht es bei der Leitfähigkeit Sinn, diese mit einem Grenzwert nach oben zu begrenzen, wohingegen eine Temperatur ggf. nach oben und nach unten sinnvoll zu begrenzen ist. Es soll auch verstanden werden, dass das erfindungsgemäße Verfahren bzw. die erfindungsgemäße Vorrichtung zur Überwachung vor allem in einem Zustand während der Behandlung eines Patienten von Vorteil sind. Beispielsweise würde eine Überwachung während eines Desinfektionsbetriebes vorteilhafterweise ausgesetzt werden. Dies beschränkt die Überwachung vorteilhafterweise auf Zeitpunkte, bei denen ein Patient einer potenziellen Gefährdung ausgesetzt ist. Ebenfalls können so an Zeitpunkten, bei denen ein Patient keiner potenziellen Gefährdung ausgesetzt ist, bspw. während einer Reinigung oder Desinfektionsphase, Sensorwerte erlaubt werden, die andernfalls zu einem Alarm oder Stopp der Anlage führen würden.

Die in FIG 3A und 3B dargestellten Anlagen unterscheiden sich durch das Vorhandensein der zweiten Pumpe 107 in der in FIG 3A dargestellten Ausführungsform. Es soll anhand FIG 3B verdeutlicht werden, dass die erdfindungsgemäße Vorrichtung auch in Systemen ohne eine solche zweite Pumpe 107 vorteilhaft eingesetzt werden kann. Insbesondere ist es möglich das Konzentrat direkt an ein Ablassventil 106 zu führen. Dabei weiß der Fachmann, dass die Rückführung hauptsächlich zur Wassereinsparung und zur Vermeidung einer sog. "Dead-End" Filtration durchgeführt wird, bei welcher die Langlebigkeit der Membranen sinkt. Alternativ zu der Pumpe 107 kann an der entsprechenden Stelle auch ein Ventil 118, insbesondere in der Form eines Nadelventils, eingesetzt werden, welches wahlweise ebenfalls durch die Steuereinrichtung 110 angesteuert werden kann. Üblicherweise ist statt oder zusätzlich zu einer Ansteuerung des Ventils 118 eine manuelle Regelung, bspw. mittels einer entsprechenden eine Handarmatur vorgesehen.

Das Abschalten der Druckpumpe 103 reicht aus, um den Umkehrosmoseprozess zu stoppen, da keine weiteren Energieerzeuger im System sind.

Die Steuerungseinheit 110 veranlasst so dann eine Regelmaßnahme, wenn entweder die Abweichung außerhalb eines Toleranzbereiches liegt oder die Sensorwerte von dem vorbestimmbaren Grenzwert des ersten Güteparameters zu stark abweichen. Eine solche Regelmaßnahme kann dabei das Erzeugen einer Statusmeldung und/oder das Steuern einer physikalischen Maßnahme umfassen, zum Beispiel eine Regelung der Pumpenleistung einer Pumpe, bevorzugt der Pumpe 103 und/oder der Pumpe 107, oder das Regeln eines Ventils, bevorzugt eines Ablassventils 106. Dies erlaubt insbesondere das Betreiben der erfindungsgemäßen Umkehrosmoseanlage mit im Durchschnitt geringer Pumpenleistung. Zwar führt eine hohe Pumpenleistung, und damit ggf. ein erhöhter Flüssigkeitsvolumenstrom, im Allgemeinen zu einer verbesserten Permeatqualität, eine geringe Pumpenleistung ist jedoch wünschenswert, insbesondere um Betriebsenergie einzusparen und somit einen umweltschonenderen und kostengünstigeren Betrieb zu ermöglichen. Jedoch kann bei geringer Pumpendrehzahl, ebenso wie bei einer Standzeit, eine ungewünschte Anreicherung im Permeat auftreten, wodurch die Leitfähigkeit ansteigt. Erfindungsgemäß kann hier als Regelmaßnahme eine Regelmaßnahme ausgelöst werden und die Pumpenleistung zeitlich begrenzt erhöht werden, wodurch sich die Anreicherung abbauen lässt und die Leitfähigkeit des Permeats zügig normalisiert werden kann. Ähnlich können kleinere Messausreiser kompensiert werden. Dabei ist es möglich ein eskalierendes Regelmaßnahmenszenario zu erstellen und bspw. einer ersten Maßnahme, z.B. der zeitlich begrenzten Erhöhung der Pumpenleistung, eine zweite Regelmaßnahme nachzuschalten, insbesondere sofern die Messwerte sich nicht normalisieren. Es ist auch daran gedacht, für die Wirkung einer ersten Regelmaßnahme ein Zeitfenster bspw. 30 s vorzugeben. Hier kann besonders vorteilhaft auch die Hierarchie der Prozessoren, nämlich eines ersten Hauptprozessors und eines zweiten Nebenprozessors genutzt werden, wobei bspw. der Hauptprozessor mit Verzögerung, bspw. dann nach 60s eine eskalierende Regelmaßnahme, im Extremfall ein Abschalten der Anlage vornimmt. Auch in der erfindungsgemäßen Vorrichtung und Umkehrosmoseanlage ist die Leitfähigkeit des Permeats 108 der bevorzugte Güteparameter, da die Leitfähigkeit ein guter Indikator für die Güte des Permeats, also insbesondere die Konzentration von Verunreinigungen, Schadstoffen, Bakterien, Viren und anderen unerwünschten Substanzen ist.

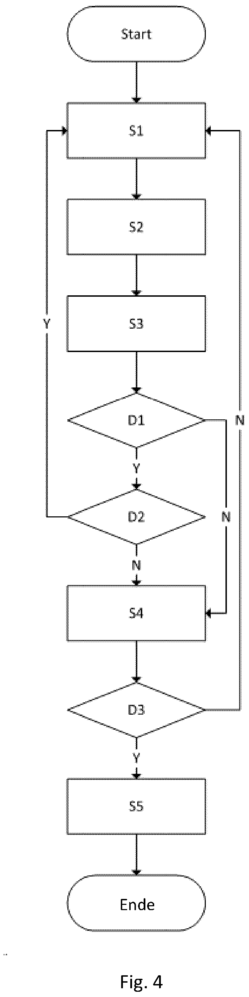

In **FIG** 4 ist ein schematisches Ablaufdiagramm für das erfindungsgemäße Verfahren gezeigt. Dabei ist das Verfahren zur Überwachung von Güteparametern von Umkehrosmose-Prozessflüssigkeiten einer Umkehrosmoseanlage, wie bspw. in FIG 3A oder FIG 3B dargestellt, geeignet. In einfachen Worten werden in dem erfindungsgemäßen Verfahren die beiden unabhängig voneinander erfassten Leitfähigkeiten gegeneinander verglichen und dürfen eine definierte Abweichung nicht überschreiten. Darüber hinaus vergleichen sowohl Hauptprozessor 115 als auch Nebenprozessor 116 die Leitfähigkeit gegen einen absoluten Grenzwert.

Als S1 dargestellt ist ein Schritt a) des Detektierens eines ersten Sensorwertes eines ersten Güteparameters eines Permeats 108 der Umkehrosmose-Prozessflüssigkeiten mittels eines ersten Güteparametersensors 109 und ein Weiterleiten des ersten Sensorwertes an einen ersten Prozessor 115 und ein Schritt b) des Detektierens eines zweiten Sensorwertes des ersten Güteparameters des Permeats 108 der Umkehrosmose-Prozessflüssigkeiten mittels eines weiteren Güteparametersensors 113 und ein Weiterleiten des zweiten Sensorwertes an einen zweiten Prozessor 116.

Mit anderen Worten erfasst hier der Hauptprozessor 115 den gesendeten Messwert des Leitfähigkeitssensors 109 und der Nebenprozessor 116 erfasst den gesendeten Messwert des Leitfähigkeitssensor 113. Diese Sequenz beinhaltet ggf. ebenfalls die Berechnung der Leitfähigkeit, falls der Sensor keinen integrierten Messumformer besitzt.

Als S2 dargestellt ist ein Schritt c) des Datentransfers zwischen dem ersten Prozessor 115 und dem zweiten Prozessor 116 mittels einer Kommunikationsschnittstelle 117. Mit anderen Worten sendet der Hauptprozessor 115 den erfassten/berechneten Leitfähigkeitswert an den Nebenprozessor 116 und umgekehrt. Der Datentransfer findet dabei über die Kommunikationsschnittstelle 117 statt.

In dem dargestellten Schritt S3 wird in einem Schritt d) die Abweichung des ersten Sensorwertes von dem zweiten berechnet. Diese Berechnung kann grundsätzlich von beiden Prozessoren ausgeführt werden. Es kann jedoch auch der Hauptprozessor 115 allein die Berechnung ausführen. Dabei wird die Abweichung bevorzugt nach der folgenden Formel berechnet.

$$\text{Formel II: Abweichung [\%]} = [\ (C1-C2)/C1\ ]*100$$

wobei C1 = Leitfähigkeit gemessen an einem ersten Güteparametersensor 109, und
wobei C2 = Leitfähigkeit gemessen an einem weiteren Güteparametersensor 113

Die Berechnung kann ebenfalls ähnlich durch den Nebenprozessor 116 ausgeführt werden und ist im Folgenden dargestellt.

$$\text{Formel III: Abweichung [\%]} = [\ (C1-C2)/C2\ ]*100$$

In einem als D1 bezeichneten Schritt e) wird die in Schritt d) festgestellte Abweichung mit einem vorbestimmten Abweichungsgrenzwert verglichen. So wird festgestellt, ob die Abweichung innerhalb eines erlaubten Bereichs liegt. Wiederum kann diese Berechnung von nur einem der beiden Prozessoren ausgeführt werden und/oder der Hauptprozessor 115 sowie der Nebenprozessor 116 vergleichen die jeweils berechnete Abweichung gegen eine zulässige Abweichung unabhängig voneinander. Der Wert der zulässigen Abweichung ist dabei bevorzugt identisch für beide Prozessoren vorbestimmt.

In dem als D2 dargestellten Schritt f) wird der erste Sensorwert und bevorzugt der zweite Sensorwert des ersten Güteparameters eines Permeats 108 der Umkehrosmose-Prozessflüssigkeiten mit einem vorbestimmten Sensorgrenzwert verglichen. Mit anderen Worten wird festgestellt, ob die Leitfähigkeiten innerhalb des erlaubten Bereichs liegen. Neben dem Vergleichen der Abweichung der Leitfähigkeiten untereinander in Schritt D1, bzw. e), wird die gemessene/- berechnete Leitfähigkeit auch gegen einen absoluten Wert verglichen. Bevorzugt vergleicht der Hauptprozessor 115 den an der Leitfähigkeitssonde 109 gemessenen Wert gegen den Grenzwert. Zusätzlich oder alternativ vergleicht der Nebenprozessor 116 den an der Leitfähigkeitssonde 113 gemessenen Wert gegen den Grenzwert. Dabei ist wiederum der vorbestimmte Sensorgrenzwert bevorzugt identisch für beide Prozessoren. Eine vorteilhafte Anwendung besteht darin, eine niedrige gemessene Leitfähigkeit gegen einen festen Grenzwert zu vergleichen, beispielsweise kleiner oder gleich 50 $\mu$S/cm, eventuell mit einer entsprechenden Toleranz von etwa +/- 5 $\mu$S/cm. Zusätzlich oder alternativ können größere Leitfähigkeiten, also beispielsweise größer als 50 $\mu$S/cm, ebenfalls mit einer Toleranz von etwa 10% Abweichung verglichen werden.

Mit dem vorliegenden erfindungsgemäßen Verfahren kann also die Funktion und die Güte des Umkehrosmoseprozessflüssigkeiten, insbesondere der Leitfähigkeit des Permeats 108, vorteilhaft überwacht werden.

Darüber hinaus kann die vorliegende Erfindung vorteilhafterweise selbst Regelmaßnahmen durchführen. Insbesondere kann in einem im Diagramm als Schritt S4 bezeichneten Schritt eine solche Regelmaßnahme vorgenommen werden, bei dem basierend auf dem Ergebnis des Vergleichens in Schritt e) bzw. D1 und/oder Schritt f) bzw. D2 von der Steuereinheit 110 die Regelmaßnahme vorgenommen wird, und wobei die Regelmaßnahme das Erzeugen einer Statusmeldung und/oder das Steuern einer physikalischen Maßnahme umfasst.

Insbesondere können so Maßnahmen zur Leitfähigkeitsreduktion vorgenommen werden.

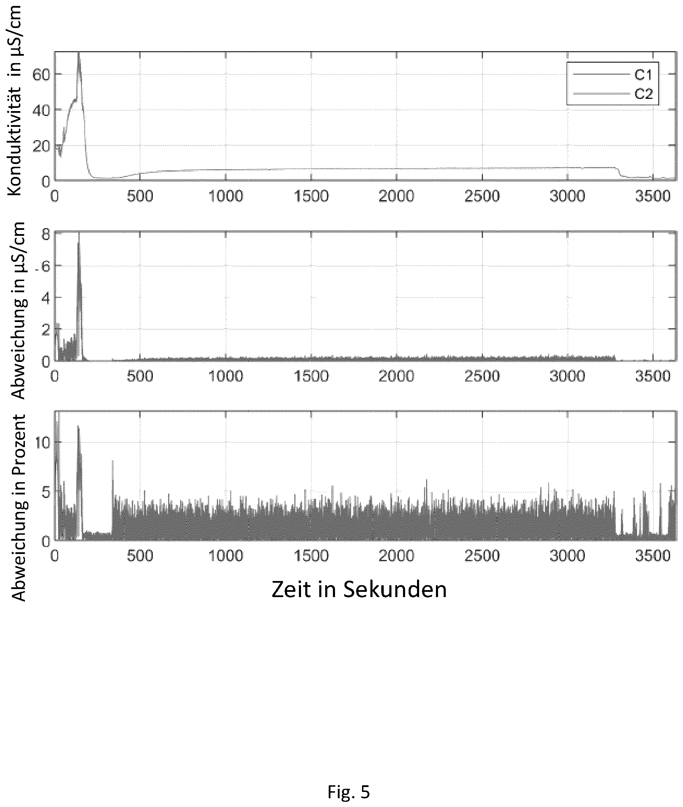

Eine solche Maßnahme ist mittels der vorliegenden Erfindung besonders wirkungsvoll und schnell, wie in FIG 5 gezeigt. Dort sind die Leitfähigkeit C1 gemessen an einem ersten Güteparametersensor 109, und die Leitfähigkeit C2, gemessen an einem weiteren Güteparametersensor 113, jeweils in $\mu$S/cm (oberer Graph), sowie die gemäß Formel II berechnete Abweichung in $\mu$S/cm (mittlerer Graph) und in [%] jeweils über die Zeit in Sekunden dargestellt. Wie unmittelbar zu erkennen, steigt die Leitfähigkeit bei etwa 100 Sekunden steil an und wird durch das erfindungsgemäße Verfahren mit einer Regelmaßnahme nach etwa 200 Sekunden ab Beginn der Messung adressiert. Dabei wird zum einen die Drehzahl der Druckpumpe 103 erhöht. Dies sorgt dafür, dass weniger Ionen durch die Membran diffundieren können, da der Arbeitsdruck gegenüber dem osmotischen Druck steigt. Daneben wird Permeat 108, in welches mehr Ionen diffundiert sind aus den Membranmodulen 104 ausgespült, was zu einer zügigen Normalisierung der Leitfähigkeit führt. Auch potenzielle Ionenansammlungen im elektrischen Feld der Leitfähigkeitssonden 109 und 113 können ggf. dadurch abtransportiert werden. Zusätzlich wurde in diesem Fall die Ausbeute verringert und das Magnetventil 106 zeitlich begrenzt geöffnet, so dass mehr Konzentrat 105 aus der Anlage geleitet wird. Dadurch wird Ionen-haltiges Konzentrat 106 durch Prozesseingangswasser 101 ersetzt. Somit wird bei gleichbleibender Filterleistung die Leitfähigkeit des Permeats 108 ebenfalls herabgesetzt. Wie aus den Graphen in Fig.5 unmittelbar ersichtlich, greifen diese physikalischen Regelmaßnahme innerhalb von nur wenigen Sekunden und die mit Güteparametersensor 109, und Güteparametersensor 113 gemessene Leitfähigkeit des Permeats 108 sinkt auf Normalwerte ab. Auch festzustellen ist, dass bei ordnungsgemäßem Betrieb und voller Funktionalität der Güteparametersensoren 109 und 113, die entsprechenden Messwerte C1 bzw. C2 nahezu identisch sind, da sich kaum Unterschiede im Kurvenverlauf zeigen.

Dabei wird, wie oben beschrieben, in Schritt D3 ein deeskalierendes Regelmaßnahmenszenario durch das vorsehen einer Verzögerungszeit verwirklicht, in dem die genannten Regelmaßnahmen, Erhöhen der Pumpenleistung und Öffnen des Ventils 106, Wirkung entfalten können, bevor es zur Abschaltung der Anlage, also dem Abschalten insbesondere der Druckpumpe 103 und der Zirkulationspumpe 107, in einem Schritt S5 kommt sofern die Messwerte sich nicht innerhalb eines vorherbestimmten Zeitfensters, der Verzögerungszeit, normalisieren. Damit können ebenfalls kurzzeitige Trends gepuffert werden. Dabei ist zu beachten, dass die Verzögerungszeit für den Fehler der Abweichung und des überschrittenen Grenzwertes gleich sein können, aber in bevorzugter Ausführung ungleich sind.

Des Weiteren sollte beachtet werden, dass die Auslösezeit für Hauptprozessor 115 und Nebenprozessor 116 gleich sein können, aber in bevorzugter Ausführung ungleich sind, wie weiter oben beschrieben. Der Hauptprozessor 115 sollte bevorzugt eine geringere Auslösezeit als der Nebenprozessor 116 haben. Weiterhin kann die Steuereinrichtung so eingerichtet werden, dass der Nebenprozessor nur bei Versagen des Hauptprozessors reagieren kann. Dabei ist es von Vorteil, dass eine Unterscheidung zwischen den beiden Fehlerarten und den beiden Prozessoren erlaubt wird. Dies ist bei der Fehleridentifikation und Fehlerbehebung von Vorteil.

Die in den Abbildungen gezeigten Ausführungsformen können sich auf bevorzugte Ausführungsformen beziehen, während alle Elemente und Merkmale, die in Verbindung mit den Ausführungsformen beschrieben werden, soweit angebracht, in Kombination mit jeder anderen Ausführungsform und jedem anderen Merkmal verwendet werden können, wie hierin diskutiert, insbesondere in Bezug auf jede andere Ausführungsform, die weiter oben besprochen wurde.

Die in der Beschreibung, den Ansprüchen, Beispielen und/oder den Abbildungen offengelegten Merkmale der vorliegenden Erfindung können sowohl einzeln als auch in jeder beliebigen Kombination davon Material zur Verwirklichung der Erfindung in verschiedenen Formen sein.

**Referenzzeichenliste**

101 - Prozesseingangswasser
102 - Vorlagetank
103 - Druckpumpe
104 - Membran(en)
105 - Konzentrat
106 - Magnetventil
107 - Zirkulationspumpe
108 - Permeat
109 - Leitfähigkeitssensor
110 - Steuerungseinheit
111 - Rücklauf
112 - Umkehrosmoseanlage
113 - Leitfähigkeitssensor
114 - zweite Steuerungseinheit
115 - Hauptprozessor
116 - Nebenprozessor
117 - Kommunikationsschnittstelle
118 - Nadelventil

**Patentansprüche**

1. Vorrichtung zur Überwachung von Güteparametern von Umkehrosmose-Prozessflüssigkeiten einer Umkehrosmoseanlage (112) umfassend

   - eine Steuerungseinheit (110) mit wenigstens einem ersten Prozessor (115) und einem zweiten Prozessor (116),
   - eine Kommunikationsschnittstelle (117), eingerichtet zum Datentransfer zwischen dem ersten Prozessor (115) und dem zweiten Prozessor (116),
   - wobei der erste Prozessor (115) eingerichtet ist zur Erfassung und Verarbeitung eines durch einen ersten Güteparametersensor (109) detektierten ersten Sensorwertes eines ersten Güteparameters eines Permeats (108) der Umkehrosmose-Prozessflüssigkeiten,
   - wobei der zweite Prozessor (116) eingerichtet ist zur Erfassung und Verarbeitung eines durch wenigstens einen weiteren Güteparametersensor (113) detektierten zweiten Sensorwertes des ersten Güteparameters des Permeats (108),
   - wobei die Steuerungseinheit (110) eingerichtet ist zum Berechnen einer Abweichung des ersten Sensorwertes von dem zweiten Sensorwert und zum Vergleichen des ersten Sensorwertes und/oder des zweiten Sensorwerts mit einem vorbestimmbaren Grenzwert des ersten Güteparameters, und
   - wobei die Steuerungseinheit (110) eingerichtet ist zum Veranlassen wenigstens einer Regelmaßnahme, bevorzugt zur Steuerung der Pumpenleistung wenigstens einer ersten Pumpe (103) oder wenigstens einer weiteren Pumpe (107), der Umkehrosmoseanlage (112),
   - wobei der erste Güteparameter bevorzugt die Leitfähigkeit des Permeats (108) ist.

2. Vorrichtung nach Anspruch 1, wobei die Steuerungseinheit (110) eingerichtet ist, abhängig von der Abweichung des ersten Sensorwertes von dem zweiten Sensorwert und/oder dem Vergleichen des ersten Sensorwertes und/oder des zweiten Sensorwerts mit einem vorbestimmbaren Grenzwert des ersten Güteparameters, die Umkehrosmoseanlage (112), insbesondere die wenigstens eine erste Pumpe (103) und/oder die wenigstens eine weitere Pumpe (107), abzuschalten, so dass die Zuführung von Permeat (108) an wenigstens ein medizinisches Endgerät, insbesondere Dialysegeräte, unterbunden wird.

3. Vorrichtung nach Anspruch 1 oder 2, wobei die Steuerungseinheit (110) eingerichtet ist, abhängig von der Abweichung des ersten Sensorwertes von dem zweiten Sensorwert und/oder dem Vergleichen des ersten Sensorwertes und/oder des zweiten Sensorwerts mit einem vorbestimmbaren Grenzwert des ersten Güteparameter des Permeats (108) der ersten Umkehrosmose-Prozessflüssigkeiten, die Pumpenleistung der wenigstens einen ersten Pumpe (103) und/oder der wenigstens einen Pumpe (107) für einen vorbestimmten Zeitraum zu erhöhen.

4. Vorrichtung nach einem der Ansprüche 1 bis 3, umfassend ein erstes printed circuit board (PCB), wobei der erste Prozessor (115) und der zweite Prozessor (116) auf dem PCB, bevorzugt mittels galvanischer Trennung, zwischen dem ersten Prozessor (115) und dem zweiten Prozessor (116) angeordnet sind und/oder um-fassend ein erstes printed circuit board (PCB) auf dem der erste Prozessor (115) angeordnet ist und ein zweites printed circuit board (PCB) auf dem der zweite Prozessor (116) angeordnet ist.

5. Vorrichtung nach einem der Ansprüche 1 bis 4, wobei die Kommunikationsschnittstelle (117) eingerichtet ist zur Kommunikation mit einer Überwachungseinheit, wobei die Überwachungseinheit außerhalb der Umkehrosmoseanlage angeordnet ist.

6. Umkehrosmoseanlage (112), umfassend

- einen Vorlagetank (102), der über eine Prozesseingangswasserzuführung (101) mit Prozesswasser versorgbar ist und weiter eine Rücklauf-Zuführung (111) umfasst, mittels der Rücklaufflüssigkeit aus einer mit wenigstens einem medizinischen Endgerät, insbesondere eines Dialysegeräts, verbundenen Ringleitung in den Vorlagetank (102) einspeisbar ist,
- wenigstens eine erste Filter-Pumpen-Einheit, mit wenigstens einer Pumpe (103) und/oder wenigstens einer Pumpe (107) und wenigstens einer Membran (104), die fluidisch mit dem Vorlagetank (102) verbunden ist, zur Erzeugung eines Permeats (108) aus der Umkehrosmose-Prozessflüssigkeiten des Vorlagetanks (102),
- eine erste Pumpe (103) und eine zweite Pumpe (107), und wobei die Steuerungseinheit (110) eingerichtet ist die Pumpenleistung der ersten Pumpe (103) und der zweiten Pumpe (107) voneinander unabhängig zu steuern,
- wobei die erste Pumpe (103) so angeordnet ist, dass die Membran (104) mittels der Pumpe (103) mit Umkehrosmose-Prozessflüssigkeiten aus dem Vorlage-tank (102) zur Erzeugung des Permeats (108) beaufschlagt werden kann, und
- eine mit der wenigstens einen Membran (104) fluidisch verbunden Permeatzuleitung angeordnet, so dass das erzeugte Permeat (108) über die Ringleitung wenigstens einem medizinischen Endgerät, insbesondere einem Dialysegerät, zuführbar ist,
- weiter umfassend eine Vorrichtung zur Überwachung der Umkehrosmoseanlage (112) nach einem der der Ansprüche 1 bis 5,
- einen ersten Güteparametersensor (109) zum Detektieren eines ersten Sensorwertes eines ersten Güteparameters des Permeats (108) der Umkehrosmose-Prozessflüssigkeiten und wenigstens einen weiteren Güteparametersensor (113) zum Detektieren eines zweiten Sensorwertes des ersten Güteparameters des Permeats (108) der Umkehrosmose-Prozessflüssigkeiten wobei der erste Güteparameter bevorzugt die Leitfähigkeit des Permeats (108) ist.

7. Umkehrosmoseanlage nach Anspruch 6, weiter umfassend wenigstens einen weiteren Güteparametersensor zur Erfassung einer Leitfähigkeit und/oder eines weiteren Güteparameters der Umkehrosmose-Prozessflüssigkeiten, insbesondere des Permeats (108), wobei der wenigstens eine weitere Güteparameter der Umkehrosmose-Prozessflüssigkeiten ausgewählt ist aus der Gruppe umfassend Leitfähigkeit, Temperatur, Volumenstrom, Druck.

8. Umkehrosmoseanlage nach Anspruch 7, wobei der wenigstens einen weiteren Güteparametersensor zur Erfassung einer Leitfähigkeit und/oder eines weiteren Güteparameters zwischen der wenigstens einen Pumpe (103) und/oder der wenigstens einen Pumpe (107) und der wenigstens einen Membran (104) angeordnet ist und/oder er wenigstens einen weiteren Güteparametersensor zur Erfassung einer Leitfähigkeit und/oder eines weiteren Güteparameters zur Messung des Güteparameters im Prozesseingangswasser (101) angeordnet ist.

9. Umkehrosmoseanlage nach einem der Ansprüche 6 bis 8, umfassend wenigstens eine weitere Filter-Pumpen-Einheit, umfassend wenigstens eine weitere Pumpe und wenigstens eine weitere Membran, wobei die wenigstens eine weitere Filter-Pumpen-Einheit in Reihe mit der wenigstens einen ersten Filter-Pumpen-Einheit fluidisch verbunden ist.

10. Verfahren zur Überwachung von Güteparametern von Umkehrosmose-Prozessflüssigkeiten einer Umkehrosmoseanlage (112), bevorzugt nach einem der Ansprüche 6 bis 9 und/oder einer Umkehrosmoseanlage (112) umfassend eine Vorrichtung nach einem der Ansprüche 1 bis 5, umfassend wenigstens die folgen-den Schritte:

- einen Schritt a) des Detektierens eines ersten Sensorwertes eines ersten Güteparameters eines Permeats

(108) der Umkehrosmose-Prozessflüssigkeiten mittels eines ersten Güteparametersensors (109) und ein Weiterleiten des ersten Sensorwertes an einen ersten Prozessor (115),
- einen Schritt b) des Detektierens eines zweiten Sensorwertes des ersten Güteparameters des Permeats (108) der Umkehrosmose-Prozessflüssigkeiten mittels eines weiteren Güteparametersensors (113) und ein Weiterleiten des zweiten Sensorwertes an einen zweiten Prozessor (116),
- einen Schritt c) des Datentransfers zwischen dem ersten Prozessor (115) und dem zweiten Prozessor (116) mittels einer Kommunikationsschnittstelle (117);
- einen Schritt d) des Berechnens einer Abweichung des ersten Sensorwertes von dem zweiten;
- einen Schritt e) des Vergleichens der in Schritt d) festgestellten Abweichung mit einem vorbestimmten Abweichungsgrenzwert;
- einen Schritt f) des Vergleichens des ersten Sensorwertes und/oder des zweiten Sensorwertes des ersten Güteparameters eines Permeats (108) der Umkehrosmose-Prozessflüssigkeiten mit einem vorbestimmten Sensorgrenzwert,
- wobei der erste Güteparameter bevorzugt die Leitfähigkeit des Permeats (108) ist.

11. Verfahren nach Anspruch 10, weiter umfassend

- einen Schritt des Vornehmens einer Regelmaßnahme bei dem basierend auf dem Ergebnis des Vergleichens in Schritt e) und/oder Schritt f) von der Steuereinheit (110) die Regelmaßnahme vorgenommen wird, und wobei die Regelmaßnahme das Erzeugen einer Statusmeldung und/oder das Steuern einer physikalischen Maßnahme umfasst.

12. Verfahren nach Anspruch 11, wobei das das Steuern einer physikalischen Maßnahme eine Regelung der Pumpenleistung einer Pumpe, bevorzugt der Pumpe (103) und/oder der Pumpe (109), und/oder das Regeln eines Ventils, bevorzugt eines Ablassventils (106), umfasst.

13. Verfahren nach einem der Ansprüche 10 bis 12, wobei das Verfahren einen Schritt des Erhöhens der Pumpenleistung der wenigstens einen ersten Pumpe (103) und/oder der wenigstens einen Pumpe (107) für einen vorbestimmten Zeitraum umfasst, wobei bevorzugt das Erhöhen abhängig ist von der in dem Schritt d) berechneten Abweichung des ersten Sensorwertes von dem zweiten Sensorwerts; und/oder dem Vergleichen in dem Schritt f) des ersten Sensorwertes und/oder des zweiten Sensorwertes des ersten Güteparameters eines Permeats (108) der Umkehrosmose-Prozessflüssigkeiten mit einem vorbestimmten Sensorgrenzwert

14. Verfahren nach einem der Ansprüche 10 bis 13, wobei Sensorwerte wenigstens eines weiteren Güteparameters der Umkehrosmose-Prozessflüssigkeiten, insbesondere des Permeats (108), mittels entsprechenden Güteparametersensoren detektiert und in Schritten c) bis f) verwendet werden, wobei der wenigstens eine weitere Güteparameter der Umkehrosmose-Prozessflüssigkeiten ausgewählt ist aus der Gruppe umfassend Leitfähigkeit, Temperatur, Volumenstrom, Druck.

15. Verfahren nach einem der Ansprüche 10 bis 14, bevorzugt Anspruch 11, wobei der vorbestimmte Abweichungsgrenzwert in Schritt e) abhängig von dem ersten Sensorwert und/oder dem zweiten Sensorwert vorbestimmt wird.

16. Computerprogramm und/oder das Computerprogramm umfassendes Speichermedium, wobei das Computerprogramm Anweisungen aufweist, die bei Ausführung durch einen Computer diesen veranlassen, das Verfahren nach einem der Ansprüche 10 bis 15 auszuführen.

Fig. 1

Fig. 2

Fig. 3A

Fig. 3B

Fig. 4

Fig. 5

Europäisches Patentamt

European Patent Office

Office européen des brevets

# EUROPÄISCHER RECHERCHENBERICHT

Nummer der Anmeldung

EP 25 16 0111

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 112 147 934 A (UNIV HARBIN SCIENCE & TECH) 29. Dezember 2020 (2020-12-29) * Absatz [0007] - Absatz [0033] * ----- | 1-5 | INV. B01D61/08 B01D61/12 |
| X | US 2017/021308 A1 (VOLKER MANFRED [DE]) 26. Januar 2017 (2017-01-26) * Absatz [0024] - Absatz [0046]; Ansprüche 1,2; Abbildung 1 * ----- | 1-16 | |
| A | DE 11 2013 005089 T5 (BAXTER HEALTHCARE SA [CH]; BAXTER INT [US]) 16. Juli 2015 (2015-07-16) * das ganze Dokument * ----- | 1-16 | |
| A | DE 196 55 228 B4 (AKSYS LTD [US]) 18. September 2008 (2008-09-18) * das ganze Dokument * ----- | 1-16 | |
| A | DE 43 31 102 A1 (WALTHER MEDIZIN TECHNIK GMBH [DE]) 16. März 1995 (1995-03-16) * das ganze Dokument * ----- | 1-16 | RECHERCHIERTE SACHGEBIETE (IPC) B01D C02F |

Der vorliegende Recherchenbericht wurde für alle Patentansprüche erstellt

| Recherchenort | Abschlußdatum der Recherche | Prüfer |
|---|---|---|
| München | 24. Juni 2025 | Kukolka, Florian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 16 0111

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-06-2025

| Im Recherchenbericht angeführtes Patentdokument | Datum der Veröffentlichung | Mitglied(er) der Patentfamilie | Datum der Veröffentlichung |
|---|---|---|---|
| CN 112147934 A | 29-12-2020 | KEINE | |
| US 2017021308 A1 | 26-01-2017 | DE 102014017404 A1 | 25-05-2016 |
| | | EP 3056257 A1 | 17-08-2016 |
| | | US 2017021308 A1 | 26-01-2017 |
| DE 112013005089 T5 | 16-07-2015 | AU 2013334902 A1 | 30-04-2015 |
| | | CA 2889059 A1 | 01-05-2014 |
| | | DE 112013005089 T5 | 16-07-2015 |
| | | GB 2522161 A | 15-07-2015 |
| | | GB 2551084 A | 06-12-2017 |
| | | HK 1212765 A1 | 17-06-2016 |
| | | MX 380587 B | 12-03-2025 |
| | | US 2014110340 A1 | 24-04-2014 |
| | | US 2015335808 A1 | 26-11-2015 |
| | | WO 2014066255 A1 | 01-05-2014 |
| DE 19655228 B4 | 18-09-2008 | AU 698545 B2 | 29-10-1998 |
| | | AU 4977996 A | 04-09-1996 |
| | | CA 2168629 A1 | 14-08-1996 |
| | | CA 2271583 A1 | 14-08-1996 |
| | | CA 2271586 A1 | 14-08-1996 |
| | | CA 2271595 A1 | 14-08-1996 |
| | | CA 2271598 A1 | 14-08-1996 |
| | | DE 19605260 A1 | 28-11-1996 |
| | | DE 19655223 B4 | 06-03-2008 |
| | | DE 19655224 B4 | 16-11-2006 |
| | | DE 19655225 B4 | 06-03-2008 |
| | | DE 19655226 B4 | 15-01-2009 |
| | | DE 19655228 B4 | 18-09-2008 |
| | | DE 19655230 B4 | 02-04-2009 |
| | | GB 2299026 A | 25-09-1996 |
| | | GB 2310602 A | 03-09-1997 |
| | | GB 2310613 A | 03-09-1997 |
| | | GB 2310614 A | 03-09-1997 |
| | | GB 2310615 A | 03-09-1997 |
| | | GB 2310616 A | 03-09-1997 |
| | | GB 2310617 A | 03-09-1997 |
| | | GB 2310618 A | 03-09-1997 |
| | | GB 2320209 A | 17-06-1998 |
| | | GB 2320210 A | 17-06-1998 |
| | | JP H09618 A | 07-01-1997 |
| | | JP 3802493 B2 | 26-07-2006 |
| | | JP 3802494 B2 | 26-07-2006 |
| | | JP 3802495 B2 | 26-07-2006 |
| | | JP 3802496 B2 | 26-07-2006 |

EPO FORM P0461

Für nähere Einzelheiten zu diesem Anhang : siehe Amtsblatt des Europäischen Patentamts, Nr.12/82

Seite 1 von 2

## ANHANG ZUM EUROPÄISCHEN RECHERCHENBERICHT
## ÜBER DIE EUROPÄISCHE PATENTANMELDUNG NR.

EP 25 16 0111

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-06-2025

| Im Recherchenbericht angeführtes Patentdokument | Datum der Veröffentlichung | Mitglied(er) der Patentfamilie | Datum der Veröffentlichung |
|---|---|---|---|
| | | JP 3802497 B2 | 26-07-2006 |
| | | JP 3834552 B2 | 18-10-2006 |
| | | JP 3834553 B2 | 18-10-2006 |
| | | JP 2002503115 A | 29-01-2002 |
| | | JP 2003199819 A | 15-07-2003 |
| | | JP 2003199821 A | 15-07-2003 |
| | | JP 2003235961 A | 26-08-2003 |
| | | JP 2003235963 A | 26-08-2003 |
| | | JP 2003235965 A | 26-08-2003 |
| | | JP 2003235966 A | 26-08-2003 |
| | | JP 2003245344 A | 02-09-2003 |
| | | JP 2004000479 A | 08-01-2004 |
| | | US 5591344 A | 07-01-1997 |
| | | US 5630935 A | 20-05-1997 |
| | | US 5645734 A | 08-07-1997 |
| | | US 5651893 A | 29-07-1997 |
| | | US 5658456 A | 19-08-1997 |
| | | US 5670050 A | 23-09-1997 |
| | | US 5674390 A | 07-10-1997 |
| | | US 5674397 A | 07-10-1997 |
| | | US 5674404 A | 07-10-1997 |
| | | US 5690821 A | 25-11-1997 |
| | | US 5690831 A | 25-11-1997 |
| | | US 5702606 A | 30-12-1997 |
| | | US 5705066 A | 06-01-1998 |
| | | US 5707086 A | 13-01-1998 |
| | | US 5714060 A | 03-02-1998 |
| | | US 5716531 A | 10-02-1998 |
| | | US 5725776 A | 10-03-1998 |
| | | US 5762782 A | 09-06-1998 |
| | | US 5783072 A | 21-07-1998 |
| | | US 5863421 A | 26-01-1999 |
| | | WO 9625214 A1 | 22-08-1996 |
| DE 4331102 A1 | 16-03-1995 | KEINE | |

EPO FORM P0461

Für nähere Einzelheiten zu diesem Anhang : siehe Amtsblatt des Europäischen Patentamts, Nr.12/82

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202024101009 U1